(19) Europäisches Patentamt / European Patent Office / Office européen des brevets

(11) **EP 4 631 928 A1**

(12) **EUROPEAN PATENT APPLICATION**

(43) Date of publication:
**15.10.2025 Bulletin 2025/42**

(21) Application number: **24315143.8**

(22) Date of filing: **10.04.2024**

(51) International Patent Classification (IPC):
**C07C 51/47** *(2006.01)*     **C07C 51/43** *(2006.01)*
**C07C 59/06** *(2006.01)*

(52) Cooperative Patent Classification (CPC):
(C-Sets available)
**C07C 51/43; C07C 51/47**     (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Metabolic Explorer**
**63360 Saint Beauzire (FR)**

(72) Inventors:
• **Colomb, Cédric**
  **63720 ENNEZAT (FR)**
• **Teissier, Mathieu**
  **63111 DALLET (FR)**
• **Cellier, Clément**
  **51420 WITRY LES REIMS (FR)**

(74) Representative: **Regimbeau**
**20, rue de Chazelles**
**75847 Paris Cedex 17 (FR)**

(54) **METHOD OF PURIFICATION OF GLYCOLIC ACID, COMPOSITION AND USE THEREOF**

(57) The present invention relates to a process for the extraction and purification of glycolic acid from a fermentation broth, comprising at least the successive steps of:
a) Filtrating the fermentation broth,
b) Passing said filtered fermentation broth through ion exchange resins, to obtain a glycolic acid solution,
c) Concentrating said glycolic acid solution with successive steps of evaporation and nanofiltration, to obtain a concentrated solution,
d) Crystallizing glycolic acid by cooling of the concentrated solution,

The present invention further concerns a cosmetic composition and a use thereof.

**EP 4 631 928 A1**

(52) Cooperative Patent Classification (CPC): (Cont.)

C-Sets
**C07C 51/43, C07C 59/06;**
**C07C 51/47, C07C 59/06**

## Description

### Field of the invention

The present invention relates to a method of purification of glycolic acid from a fermentation broth. It also relates to the bio-sourced glycolic acid composition such as obtained, and to uses thereof.

### Background of the invention

Glycolic acid (chemical formula: $HOCH_2COOH$, CAS Number: 79-14-1), and its conjugate base glycolate, is the simplest member of the alpha-hydroxy acid family of carboxylic acids.

Glycolic acid has dual functionality with both alcohol and moderately strong acid functional groups on a very small molecule. Its properties make it ideal for a broad spectrum of consumer and industrial applications, including use in water well rehabilitation, leather industry, oil and gas industry, laundry and textile industry, cleaning products, and as a component in personal care products. Glycolic acid can also be used in polymers production, including poly(glycolic acid) or poly(lactic-co-glycolic acid), as an additive in plastics or as a food preservative.

Glycolic acid is widely used in the cosmetics industry, in dermatological, medical and cosmetic skin care techniques. Indeed, it has a particular ability to penetrate the skin and accelerate skin cell renewal by means of peels. Glycolic acid helps improving skin texture and appearance, by eliminating wrinkles, acne and/or hyperpigmentation. Glycolic acid reacts with the top layer of the epidermis, by weakening the lipid bonds holding dead skin cells together: this eliminates the skin top layer, revealing the lower layer that is younger, tonic and glowing.

Currently, more than 50,000 tons of glycolic acid are consumed annually worldwide.

Although glycolic acid occurs naturally as a trace component in sugarcane, beets, grapes and fruits, it is synthetically produced by two main ways. Glycolic acid can be produced by a catalyzed reaction of formaldehyde with synthesis gas (carbonylation of formaldehyde). Glycolic acid can also be produced from methoxyacetic acid, a process which has the advantage to be "formaldehyde free".

With both these synthesis ways, the produced glycolic acid solution contains by-products such as unconverted formaldehyde or unreacted methoxyacetic acid, which are both considered to be CMR (Carcinogenic, Mutagenic and Reprotoxic) agents. Further, chemically synthetized glycolic acid compositions may contain by-products having side-effects such as skin irritation.

Other technologies for producing glycolic acid, more respectful of the environment and ensuring a high quality product free of toxic by-products, have been described in the literature or in patent applications.

Enzymatic production of glycolic acid using glycolonitrile as the starting material and a nitrilase enzyme is known in the art.

Further, methods and microorganisms for producing glycolic acid by fermentation from renewable resources, wherein carbohydrates are converted into glycolic acid by fermentation, are disclosed in international applications WO 2007/140816, WO 2007/141316, WO 2010/108909, WO 2011/036213, WO 2011/157728, WO 2012/025780, CN105647844A, CN106011185A, WO 2016/079440 and WO2018/138240 for methods using *Escherichia coli* strains, and in international applications WO 2013/050659, WO 2014/162063 and WO 2016/193540 for methods using *Saccharomyces cerevisiae* or *Kluyveromyces lactis* strains.

The main drawback of these methods of fermentative production is the difficulty to purify the produced glycolic acid from the fermentation broths. Therefore, optimization of glycolic acid extraction and purification from fermentation broths has been investigated. In particular, there is a need to implement a purification process allowing the obtention of a bio-sourced glycolic acid composition with a minimum of by-products, and therefore a high level of purity.

Two French patents disclose purification processes from fermentation broths of *Escherichia coli* strains. The patent FR2974803B1 discloses a purification process based on passages of the clarified broth through exchange resins and distillation after a step of concentration. The patent FR2974804B1 discloses a purification process based on passages of the clarified broth through exchange resins, bipolar electrodialysis, nanofiltration and distillation after a step of concentration.

Both French patents mention that the step of distillation is usually difficult, since at high temperature, glycolic acid tends to poly-condensate into glycolic acid oligomers. These patents propose specific conditions of distillation, performed during a short time (less than 5 minutes), to overcome this difficulty and collecting a glycolic acid composition without oligomers. Nevertheless, the obtained glycolic acid compositions contain other organic acids (citric acid, acetic acid, ...) and glucose. None of these patents discloses a process including a step of crystallization.

The international application WO 2006/069129 discloses a process of glycolic acid purification from an aqueous solution of ammonium glycolate produced by an enzymatic process. This process involves evaporation of water to obtain crystals, and then heating up the crystals at a temperature of 100°C to 140°C under vacuum, to remove ammonium. The major drawback of this technique is that the obtained composition comprises glycolic acid oligomers.

The European patent EP1947079B1 discloses a process of glycolic acid purification from a fermentation broth comprising 3 to 5 steps in any order, including electrodialyzing, water-splitting electrodialysis, cooling crystallization, treatment with an activated carbon and passage through an ion exchange resin. The fermentation broth contains ethylene glycol that is the substrate converted into glycolic acid. Purification is considered to be satisfying when the molar ratio of ethylene glycol in the final composition is less than 0.1% and the concentration of ammonium ion is less than 15 ppm. The major drawback of this technique is also the nature and quantity of by-products present in the final composition.

The present invention addresses the above needs, providing an improved process of extraction and purification of glycolic acid from a fermentation broth, which can be implemented on an industrial scale.

**Brief description of the invention**

This process of extraction and purification of glycolic acid from a fermentation broth comprises at least the following successive steps:

a) Filtrating the fermentation broth,
b) Passing said filtered fermentation broth through ion exchange resins, to obtain a glycolic acid solution,
c) Concentrating said glycolic acid solution with successive steps of evaporation and nanofiltration, to obtain a concentrated solution,
d) Crystallizing glycolic acid by cooling of the concentrated solution.

The present invention also provides a composition of bio-sourced glycolic acid, comprising as main components glycolic acid, succinic acid and uracil.

The present invention also concerns the use of a composition as described above for cosmetic applications, in particular as a moisturizing agent or as an exfoliating agent.

Finally, another aspect of the present invention relates to a cosmetic composition comprising, as an active agent, a composition as described above.

**Brief description of the figures**

**Figure 1.** Description of the recycling fluxes containing glycolic acid at the steps c and d. The diafiltrate obtained after substep (c2) and the mother liquor obtained after step (d) are recycled in the process.

**Figure 2.** Hydration of the superficial layers of the epidermis obtained by application of different glycolic acid compositions: GA-METEX according to the invention, and GA-CONTROL commercially available.

2A: Immediate effect. Measures are performed 10 minutes after the application.

2B: Measures are performed after 14 days of daily application.

**Figure 3.** TransEpidermal Water Loss (TEWL) measured after application of different glycolic acid compositions: GA-METEX according to the invention, and GA-CONTROL commercially available.

3A: Immediate effect. Measures are performed 10 minutes after the application.

3B: Effect observed from T7 day to T14 day.

**Figure 4.** Sebum secretion measured after application of different glycolic acid compositions: GA-METEX according to the invention, and GA-CONTROL commercially available.

4A: Immediate effect. Measures are performed 10 minutes after the application.

4B: Sebum secretion measured after 14 days of daily application.

**Figure 5.** Usage test results measured after application on the skin of different glycolic acid compositions: GA-METEX according to the invention (in black), and GA-CONTROL commercially available (in grey).

5A: Evaluation of uniformity and nourishing of the skin after 7 days of daily treatments.

5B: Evaluation of smoothness, lifting, luminosity, fineness, and firmness parameters of the skin, measured at the 7th and the 14th days of daily treatment.

**Detailed description of the invention**

Before describing the present invention in detail, it is to be understood that the invention is not limited to particularly exemplified processes and may, of course, vary. Indeed, various modifications, substitutions, omissions, and changes may be made without departing from the scope of the invention. It shall also be understood that the terminology used herein is for the purpose of describing particular embodiments of the invention only and is not intended to be limiting.

All publications, patents and patent applications cited herein, whether supra or infra, are hereby incorporated by reference in their entirety. Furthermore, the practice of the present invention employs, unless otherwise indicated, conventional microbiological and molecular biological techniques that are within the skill of the art. Such techniques are well-known to the skilled person and are fully explained in the literature.

Unless defined otherwise, all technical and scientific terms used herein have the same meanings as are commonly understood by one of ordinary skill in the art to which this invention belongs. Although any materials and methods similar or equivalent to those described herein can be used to practice or test the present invention, preferred material and methods are provided.

It must be noted that as used herein and in the appended claims, the singular forms "a," "an," and "the," include plural reference unless the context clearly dictates otherwise. Thus, for example, a reference to "a microorganism" includes a plurality of such microorganisms, and a reference to "an endogenous gene" is a reference to one or more endogenous genes, and so forth.

The terms "comprise," "contain," and "include" and variations thereof such as "comprising" are used herein in an inclusive sense, i.e., to specify the presence of the stated features but not to preclude the presence or addition of further features in various embodiments of the invention.

Genes and proteins are identified herein using the denominations of the corresponding genes in *E. coli* (e.g., *E. coli* K12 MG1655 having the Genbank accession number U00096.3) unless otherwise specified. However, in some cases use of these denominations has a more general meaning according to the invention and covers all of the corresponding genes and proteins in microorganisms.

A first aspect of the invention concerns a process for the extraction and purification of glycolic acid from a fermentation broth, comprising at least the successive steps of:

a) Filtrating the fermentation broth,
b) Passing said filtered fermentation broth through ion exchange resins, to obtain a glycolic acid solution,
c) Concentrating said glycolic acid solution with successive steps of evaporation and nanofiltration, to obtain a concentrated solution,
d) Crystallizing glycolic acid by cooling of the concentrated solution.

"Glycolic acid", also designated as hydroxyacetic acid, is a product of chemical formula $HOCH_2CO_2H$, of CAS number 79-14-1. Glycolic acid is highly soluble in water. In an aqueous solution, it may be present under several forms. First, the H+ proton of the acid function $CO_2H$ tends to separate from the radical: the negatively charged salt $HOCH_2CO_2^-$ is designated as "glycolate". Secondly, two molecules of glycolic acid are susceptible to dimerize, under a cyclic form called glycolide (CAS No. 502-97-6) or under a linear form called diglycolic acid (CAS No. 110-99-6).

In the sense of the invention, the term "glycolic acid" - also designated as "GA" in the figures and tables - includes all these forms of glycolic acid, notably the glycolate salt and the cyclic and linear dimers.

The present invention concerns extraction and purification of bio-sourced glycolic acid, being obtained from natural sources.

In the sense of the invention, "bio-sourced glycolic acid" designates glycolic acid produced by plants or microorganisms, preferably from renewable carbon sources. The term "bio-sourced glycolic acid" excludes glycolic acid obtained by chemical synthesis.

In particular, the bio-sourced glycolic acid is produced by microorganisms and is recovered from a fermentation broth.

A "fermentation broth" designates, in the sense of the invention, both the culture medium where the microorganisms producing glycolic acid are cultivated, i.e. the broth recovered at the end of the culture, and the pretreated broth ready for the implementation of the process.

The fermentation broth may indeed be pre-treated with any of the usual pre-treatment steps well known by the person skilled in the art, for example addition of anti-foam agents, adjustment of the pH, thermal deactivation using heat exchangers and/or steam injection, and/or decantation.

According to a specific embodiment, said fermentation broth results from the culture of a genetically modified microorganism expressing heterologous genes encoding enzymes involved in glycolic acid synthesis, in a nutritive medium.

According to the invention, the terms "culture", "fermentative process", "fermentative production" or "fermentation" are used interchangeably to denote the growth of microorganism. This growth is generally conducted in fermenters with an appropriate growth medium adapted to the microorganism being used.

The terms "nutritive medium", "growth medium" and "culture medium" are used interchangeably to designate a medium (e.g., a sterile, liquid media) comprising nutrients essential or beneficial to the maintenance and/or growth of the cell such as carbon sources or carbon substrates, nitrogen sources; phosphorus sources, for example, monopotassium phosphate or dipotassium phosphate; trace elements (e.g., metal salts), for example magnesium salts, cobalt salts and/or manganese salts; as well as growth factors such as amino acids and vitamins. In particular, the inorganic culture medium can be of identical or similar composition to an M9 medium (Anderson, 1946), an M63 medium or a medium such as defined by Schaefer et al. (1999).

The term "source of carbon," "carbon source," or "carbon substrate" according to the present invention refers to any carbon source capable of being metabolized by a microorganism wherein the substrate contains at least one carbon atom. According to the present invention, said source of carbon is preferably at least one carbohydrate, and in some cases a mixture of at least two carbohydrates.

The term "source of nitrogen" according to the present invention refers to any nitrogen source capable of being used by the microorganism. Said source of nitrogen may be inorganic (e.g., $(NH_4)_2SO_4$) or organic (e.g., urea or glutamate). Preferably, said source of nitrogen is in the form of ammonium or ammonia solution.

The person skilled in the art is able to define the culture conditions for the microorganisms according to the invention.

In the present context, the microorganism is preferably a bacterium, yeast or fungus.

Preferably, the microorganism is selected from the *Enterobacteriaceae*, *Clostridiaceae*, *Bacillaceae*, *Streptomycetaceae*, *or Corynebacteriaceae* family or from among yeast, more preferably from the *Saccharomycetaceae* family.

Even more preferably, the microorganism is a species of *Escherichia*, *Klebsiella*, *Thermoanaerobacterium*, *Clostridium*, *Corynebacterium* or *Saccharomyces.*

Even more preferably, said *Enterobacteriaceae* bacterium is *Escherichia coli* or *Klebsiella pneumoniae*, said Clostridiaceae bacterium is *Clostridium acetobutylicum*, said Corynebacteriaceae bacterium is *Corynebacterium glutamicum*, or said Saccharomycetaceae yeast is *Saccharomyces cerevisiae*.

Most preferably, the genetically modified microorganism is of the species *Escherichia coli*.

The term "genetically modified microorganism" refers to a microorganism or a strain of microorganism that has been genetically modified or genetically engineered. This means, according to the usual meaning of these terms, that said modified microorganism is not found in nature and is genetically modified when compared to the "parental" microorganism from which it is derived. The "parental" microorganism may occur in nature (i.e., a wild-type microorganism) or may have been previously modified. The recombinant microorganisms of the invention may notably be modified by the introduction, deletion and/or modification of genetic elements. Such modifications can be performed, for example, by genetic engineering or by adaptation, wherein a microorganism is cultured in conditions that apply a specific stress on the microorganism and induce mutagenesis.

Said genetically modified microorganism expresses heterologous genes encoding enzymes involved in glycolic acid synthesis.

A microorganism may be genetically modified to express one or more exogenous or heterologous genes so as to overexpress the corresponding gene product (e.g., an enzyme, in the present case an enzyme involved in glycolic acid synthesis). The term "heterologous gene" indicates that a gene was introduced into a microorganism wherein said gene is not naturally occurring in said microorganism. The heterologous gene may be directly integrated into the chromosome of the microorganism or be expressed extra-chromosomally within the microorganism by plasmids or vectors.

Using the references given in Genbank for known genes, those skilled in the art are able to determine the equivalent genes in other organisms, bacterial strains, yeast, fungi, mammals, plants, etc. This routine work is advantageously done using consensus sequences that can be determined by carrying out sequence alignments with genes derived from other microorganisms and designing degenerate probes to clone the corresponding gene in another organism. These routine methods of molecular biology are well known to those skilled in the art.

Genes encoding enzymes involved in glycolic acid synthesis are in particular genes encoding xylulose 5-phosphate phosphoketolase and fructose 6-phosphate phosphoketolase.

The term "phosphoketolase" typically refers to enzymes with xylulose 5-phosphate phosphoketolase activity (EC 4.1.2.9) and/or fructose 6-phosphate phosphoketolase activity (EC 4.1.2.22).

Xylulose 5-phosphate phosphoketolase activity means the activity of phosphate-consuming conversion of xylulose 5-phosphate to glyceraldehyde 3-phosphate and acetylphosphate with release of water.

Xylulose 5-phosphate phosphoketolase enzyme or its encoding gene may originate from bacteria, including lactic acid bacterium, methanol-assimilating bacterium, methane-assimilating bacterium, *Streptococcus* bacterium, and more specifically bacteria belonging to the genera *Acetobacter, Bifidobacterium, Lactobacillus, Thiobacillus, Streptococcus, Methylococus, Butyrivibrio, Fibrobacter*, and/or yeast belonging to the genera *Candida, Rhodotorula, Rhodosporidium, Pichia, Yarrowia Hansenula, Kluyveromyces Saccharomyces, Trichosporon, Wingea* or the like.

Fructose 6-phosphate phosphoketolase activity means the activity of phosphate-consuming conversion of fructose 6-phosphate to erythrose 4-phosphate and acetylphosphate with release of water.

Fructose 6-phosphate phosphoketolase enzyme or its encoding gene may originate from bacteria that belong to the genera *Acetobacter, Bifidobacterium, Chlorobium, Brucella, Methylococus, Gardnerella*, and/or yeast belonging to *Rhodotorula, Candida, Saccharomyces* or the like.

It has been reported that some enzymes catalyse both activities of xylulose 5-phosphate phosphoketolase and fructose 6-phosphate phosphoketolase, such as Xfp from *Bifidobacterium animalis* (see WO 2006/016705 and WO 2016/044713) or from *Bifidobacterium lactis* (Meile *et al.,* 2001).

According to an embodiment of the invention, phosphoketolase encoding genes are chosen among: *xfp* gene from *Bifidobacterium animalis* (WO 2006/016705 and WO 2016/044713), *xfp* gene from *Bifidobacterium lactis* (Meile et al., 2001), *xpkA* from *Lactobacillus pentosus* (Posthuma et al., 2002) or their homologous genes: *xpk1* gene from *Lactobacillus plantarum, xpk2* gene from *Lactobacillus plantarum, xpk* gene from *Streptococcus agalactiae* NEM316, ptk gene from *Lactococcus lactis subsp. lactis, xpk* gene from *Lactobacillus johnsonii, xpk* gene from *Lactobacillus acidophilus, xfp* gene from *Bifidobacterium longum, xfp* gene from *Chlorobium tepidum, xfp* gene from *Brucella suis*, and *xfp* gene from *Brucella abortus.*

Preferentially, the microorganism overexpresses *xfp* gene from *Bifidobacterium lactis, xfp* gene from *Bifidobacterium animalis*, or *xpkA* from *Lactobacillus pentosus*, or one of their homologous genes.

In a preferred embodiment of the invention, the modified microorganism further overexpresses ycdW and/or *yiaE* gene in order to increase the conversion of glyoxylic acid into glycolic acid, as disclosed in patent application WO 2007/141316.

In addition of the expression of heterologous genes encoding enzymes involved in glycolic acid synthesis, the genetically modified microorganism may present further genetic modifications.

In a specific embodiment of the invention, in the microorganism used for producing glycolic acid into the fermentation broth, the expression of at least one gene chosen among *aceB, glcB, gcl* and *eda* is attenuated. As disclosed in international application WO 2007/141316, deletion of at least one gene chosen among aceB encoding malate synthase, *glcB* encoding malate synthase, gel encoding glyoxylate carboligase and *eda* encoding 2-keto-3-deoxygluconate 6-phosphate aldolase leads to a decrease of the conversion of glyoxylate allowing an accumulation of glyoxylate which will be further converted into glycolic acid.

More preferably, said microorganism used for producing glycolic acid into the fermentation broth is further modified as disclosed in patent applications WO 2010/108909, WO 2011/036213, WO 2011/157728, WO 2012/025780 with:

- attenuation of the genes *glc*DEFG encoding glycolate oxidase and/or *aldA* encoding glycoaldehyde dehydrogenase leading to the inability to substantially metabolize glycolate;

- increase of the glyoxylate pathway flux, obtained in particular by the attenuation of the genes *icd* encoding isocitrate dehydrogenase, *aceK* encoding Icd kinase-phosphatase, *pta* encoding phospho-transacetylase, *ackA* encoding acetate kinase, poxB encoding pyruvate oxidase, *icl*R *or fadR* encoding glyoxylate pathway repressors, and/or by the overexpression of the gene *aceA* encoding isocitrate lyase;

- increase of the availability of NADPH, obtained in particular by the attenuation of the genes *pgi, udh*A, *edd.*

In a specific embodiment, the genetically modified microorganism that is cultivated to produce a fermentation broth containing glycolic acid is one of those disclosed in the international application WO2018/138240. In particular, said microorganism is from the *Escherichia coli* species, and presents at least the following genetic modifications:

- Expression of at least one gene chosen among *aceB, glcB, gcl* and *eda* is attenuated, and
- Expression of at least one gene encoding xylulose 5-Phosphate phosphoketolase and/or fructose 6-Phosphate phosphoketolase is enhanced.

The obtained fermentation broth, containing glycolic acid, may be pre-treated with any of the usual pre-treatment steps, well known by the person skilled in the art, for example thermal treatment, addition of anti-foam agents, adjustment of the pH, and/or decantation.

Pretreatment conditions applied to glycolic acid fermentation broth are sufficient to obtain a F0 value that a person skilled in the art can calculate and can be able to reproduce. The term F0 value designates the equivalent exposure time at 121.1 °C to that of the actual exposure time at a variable temperature calculated with a temperature coefficient of the destruction of 10°C. A minimum F0 of 0.5 is applied so that viability is lower than 0.1 CFU (Colony Forming Unit)/mL in the pretreated broth.

### *Process of extraction and purification of glycolic acid*

The process according to the invention comprises at least the successive steps of:

a) Filtrating the fermentation broth,
b) Passing said filtered fermentation broth through ion exchange resins, to obtain a glycolic acid solution,
c) Concentrating said glycolic acid solution with successive steps of evaporation and nanofiltration, to obtain a concentrated solution,
d) Crystallizing glycolic acid by cooling of the concentrated solution.

As mentioned above, in a specific embodiment of the invention, the fermentation broth is pre-treated, preferably by thermal treatment, more preferably by a thermal desactivation step using heat exchangers and/or steam injection.

In a specific embodiment of the invention, the process further comprises a step of clarification of the fermentation broth.

A step of clarification designates a step of removing the insoluble organic impurities from said fermentation medium. The clarification of the medium is carried out by any method known as such by those skilled in the art, which method is chosen, for example, from the group consisting of heating, flocculation, decanting, membrane techniques (microfiltration, ultrafiltration, diafiltration, nano-filtration and reverse osmosis) and centrifugation.

As is well known by the person skilled in the art, filtration designates a physical separation process that separates solid matter and fluid from a mixture using a membrane that has a complex structure through which only the fluid can pass. Solids and solutes of high molecular weight are retained in the so-called retentate, while water and low molecular weight solutes pass through the membrane in the permeate (also called filtrate).

There are many different types of filtrations known by those skilled in the art, for example tangential filtration, dynamic crossflow filtration, nanofiltration of acid, etc. "Tangential filtration" is a rapid and efficient method for the separation and purification of biomolecules by utilizing ultrafiltration membranes. "Nanofiltration of acid" (NFA) is an efficient method for purification of acids using acid resistant organic membranes due to the low pH of the feeding solution.

Preferably, this step of clarification is carried out by ultrafiltration.

In the sense of the invention, the term "ultrafiltration" designates a type of membrane filtration in which forces such as pressure or concentration gradients lead to a separation through a semipermeable membrane.

In a specific embodiment of the invention, the clarification step is performed with a ceramic or organic membrane.

Ceramic membranes are artificial membranes made from inorganic materials.

Advantageously, and by contrast with organic membranes, ceramic membranes can be used in separations where aggressive media (acids and/or strong solvents) are present. They have an excellent thermal stability, which makes them usable in high-temperature filtration operations.

Organic membranes are made of polymeric organic materials (polyethylene terephthalate, polysulfone, polypropylene, cellulose acetate, polylactic acid). They are widely used for ultrafiltration, nanofiltration and reverse osmosis applications.

### *Step (a) - Filtration of the fermentation broth*

Step (a) involves filtrating the fermentation broth, optionally previously pretreated, and/or optionally previously clarified.

In a specific embodiment, step (a) is performed by nanofiltration.

In the sense of the invention, the term "nanofiltration" designates a type of filtration that uses a membrane with nanometer sized pores, through which particles smaller than about 1 to 10 nanometers pass through the membrane. Nanofiltration membranes have pore sizes smaller than those used in microfiltration and ultrafiltration.

After step(a), the obtained product is hereby designated as a "filtered fermentation broth" or "nanofiltration permeate".

### *Step (b) - Passage through ion exchange resins*

The filtered fermentation broth is passed through at least one ion exchange resin, preferably at least two ions exchange resins.

In a specific embodiment, in step (b), the filtered fermentation broth is successively passed through a strong cation exchange resin and a weak anion exchange resin.

The first passage through a strong cation exchange resin (step b1) is preferably performed on a macroporous exchange resin. The term "strong" refers to the force of attraction between the cations and the resin. In the cationic resin, the glycolate ions are not adsorbed and are converted to their acidic form (glycolic acid).

Examples of strong cationic resins are bead-like products which have a sulfonic acid group in a cross-linked styrene frame. The person skilled in the art knows, for example, the commercially available exchange resins designated as POROS™ XS from ThermoFisher and DIAION™ PK or LK from Mitsubishi chemical.

The second passage through a weak anion exchange resin (step b2) is preferably performed on a gel exchange resin. The term "weak" refers to the force of attraction between the resin and the anions to be removed from the glycolic acid solution. Only molecules with a lower pKa than glycolic acid's one are removed at this step (such as oxalic and orotic acids).

After these steps b1 and b2, the obtained product is designated as "the glycolic acid solution". It contains glycolic acid, acetic acid, glucose, succinic acid, uracil and formic acid.

### Step (c) - Concentration of glycolic acid

Step (c) corresponds to the concentration of glycolic acid into the aqueous solution containing it. This concentration step may be achieved by any mean known by the person skilled in the art.

In a particular embodiment of the invention, in step (c), the glycolic acid solution is successively:

- (c1) concentrated by water evaporation,
- (c2) nanofiltered, and
- (c3) concentrated with a rectification column, including addition of stripping water.

The first substep (c1) corresponds to water evaporation of the aqueous solution. The most usual mean for water evaporation is the heating of the solution up to 100°C, but it can be performed by any other mean known by the person skilled in the art, such as vacuum evaporation. This substep allows to obtain a glycolic acid solution concentrated to a range between 10 to 40% w/w (weight of glycolic acid / total weight of the solution).

The second substep (c2) is a step of nanofiltration, intended for removing impurities from the glycolic acid solution, mainly glucose and partially succinic acid.

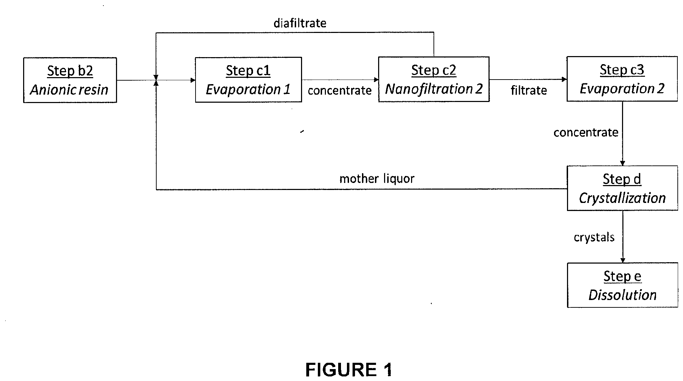

At this step, a diafiltration can be applied to improve glycolic acid recovery and the diafiltrate is recycled in step c1. (see figure 1)

The third substep (c3) is a step of concentration using a rectification column, including addition of stripping water. This substep (c3) allows removing species more volatile than glycolic acid, i.e., organic acids such as acetic acid and formic acid.

A rectification column is used in a fractional distillation process, based on successive distillation steps. Rectification columns use the difference in volatility of the components in a mixture to separate them. To improve separation, a large exchange surface between the gas and liquid phases is provided. As the solution to be purified is heated, its vapors rise into the column and condense on the walls and the surfaces of the column. Two heat exchangers provide the energy required for separation: a boiler at the bottom of the column, where the mixture is heated to boiling point, and a condenser at the top of the column, which liquefies the vapors to recover the purified product in liquid form.

The "stripping water" is added to the solution to be purified, and re-distillated to recover the light impurities in distillate while rectification limits the loss of glycolic acid in distillate.

The product obtained after performing all substeps of step (c) is designated as a "concentrated solution of glycolic acid".

The glycolic acid is concentrated at least at 55% w/w content. A "ratio w/w" or "w/w" or "w/w content" means that the ratio is in weight of glycolic acid over the total weight of the concentrated solution.

Preferably the glycolic acid represents between 55% and 75% in weight of the concentrated solution obtained after step (c).

### Step (d) - Crystallization

Step (d) corresponds to the crystallization of glycolic acid, by cooling of the concentrated solution of glycolic acid obtained in step (c).

In a particular embodiment, in step (d), the temperature of the concentrated solution is lowered to a temperature ranging from 5°C to -25°C, preferably ranging from 0°C to -20°C, more preferentially from -5°C to -20°C, and in particular ranging from -10°C to -20°C.

At the end of the cooling step, a slurry mixture is obtained. The term "slurry" designates a mixture of denser solids suspended in liquid, usually water.

The slurry mixture is treated by centrifugation to separate and recover crystals of glycolic acid from the mother liquor. The term "mother liquor" designates the solution remaining after the removal of crystals.

At this step, mother liquor could be recycled in step c1. (see figure 1)

At the end of centrifugation step, crystals of glycolic acid are obtained: they contain at least 85%, 86%, 87%, 88%, 89%, 90%, 91%, 92%, 93%, 94% or 95% of glycolic acid; preferentially, these crystals contain about 95% of glycolic acid and about 5% of by-products and/or residual water.

### Step (e) - Dissolution of glycolic acid crystals

In a particular embodiment of the invention, the process further comprises a step (e) of dissolution of glycolic acid crystals into an aqueous buffer, and of polishing with an absorbent, preferably activated carbon.

This step can be divided into two substeps (e1) and (e2) which are performed successively in the order (e1) then (e2).

Step (e1) corresponds to the dissolution of crystals of glycolic acid, such as obtained at the end of step (d), into an aqueous buffer. This dissolution is preferentially performed under agitation. The aqueous buffer is preferentially water. At this step, it is possible to determine APHA/Platinum-Cobalt color for the obtained glycolic acid solution.

The APHA/Platinum-Cobalt color scale is described in ASTM D1209 "Standard Test Method for Color of Clear Liquids (Platinum-Cobalt Scale)". It is used to evaluate pollution levels in water or wastewater along with determining product quality and impurities. It is also used for color determination of liquid chemicals such as alcohols, organic acids, solvents, that are liquid at room temperature.

Usually, a spectrophotocolorimeter Lovibond is used to determine the APHA/Platinum-Cobalt color value.

Standard APHA color value for cosmetic glycolic acid solution is lower than 15 mg/L Pt-Co. The glycolic acid composition according to the invention presents an APHA color value comprised between 0 and 15 mg/L Pt-Co. Preferably, the APHA color value is comprised between 0 and 10 mg/L Pt-Co, or between 0 and 5 mg/L Pt-Co.

Step (e2) corresponds to the "polishing" of the glycolic acid. The person skilled in the art knows that process, which involves addition of an adsorbent to the solution.

Adsorbents are in the form of spherical pellets, rods, moldings, or monoliths. They have high abrasion resistance, high thermal stability and small pore diameters. Most industrial adsorbents fall into one of the following classes:

- Oxygen-containing compounds, including materials such as silica gel, limestone (calcium carbonate) and zeolites;
- Carbon-based compounds, including materials such as activated carbon and graphite;
- Polymer-based compounds, including resins.

In the context of the invention, the used adsorbent is chosen among resin or carbon-based compounds, and is preferably activated carbon.

This step of polishing allows to remove impurities in the final glycolic acid composition. In particular, traces of formaldehyde or its precursors that may be present are removed with this polishing step. Advantageously, at the end of step (e2), the concentration of formaldehyde is lower than 5 ppm in the final bio-sourced glycolic acid composition.

### Crystals and compositions of bio-sourced glycolic acid

The present invention also concerns a composition of bio-sourced glycolic acid, comprising as main components glycolic acid, succinic acid and uracil.

The bio-sourced glycolic acid composition advantageously contains a minimum of by-products, and therefore the glycolic acid presents a high level of purity.

The composition comprising bio-sourced glycolic acid is, by itself, bio-sourced.

In the sense of the invention, the term "by-products" designates all chemical entities present in the glycolic acid composition, which are detectable (i.e., in a quantity superior to 5 ppm) and which are of a different nature than glycolic acid.

Specifically, by-products include nucleobases, hydroxy acids, organic acids, and ions.

More specifically, by-products are chosen among the group consisting of: uracil, succinic acid, acetic acid, formic acid, glyoxylic acid, malic acid, citric acid, lactic acid, methoxyacetic acid, chloride, sulfate, ammonia, calcium, magnesium, potassium, sodium, and any of their mixtures.

In the sense of the invention, the sentence "a minimum of by-products" means both that:

- a low number of detectable by-products is present in the glycolic acid composition (typically, less than 20, 15, 10, 5 or even only 2 by-products); and

- each of these detectable by-products is present in a low quantity (typically, less than 5000, 1000, 500, 200, 100, 80, 50 ppm or even less than 20 ppm).

The pseudo-unit « ppm » corresponds to "parts-per-million" ($10^{-6}$). It is used to describe small values of miscellaneous dimensionless quantities, for example mole fraction or mass fraction. Since these fractions are quantity-per-quantity measures, they are pure numbers with no associated units of measurement. However, in aqueous solutions, it is considered that 1 ppm corresponds to about 1 mg/kg.

The major by-product of the composition of the invention is succinic acid. Another observed by-product is uracil.

In a specific embodiment of the invention, in the composition of bio-sourced glycolic acid:

- the succinic acid concentration ranges from 500 and 5000 ppm, preferably from 1000 and 3000 ppm, and

- the uracil concentration is lower than 500 ppm, preferably lower than 200 ppm, or even lower than 100 ppm.

In another embodiment of the invention, the composition comprises uracil and succinic acid, and further comprises as by-product glyoxylic acid, preferably in a concentration lower than 200 ppm, preferably lower than 150 ppm.

These three chemical entities are non-toxic and do not present any health risk for a use in humans.

In a specific embodiment, the composition of bio-sourced glycolic acid does not comprise glucose. In another specific embodiment, the composition of bio-sourced glycolic acid does not comprise oligomers of glycolic acid, but only glycolic acid dimers (linear and/or cyclic). In another embodiment, the composition of bio-sourced glycolic acid does not comprise methoxyacetic acid.

In a specific embodiment, the bio-sourced glycolic acid composition does not comprise glucose, oligomers of glycolic acid, and neither methoxyacetic acid.

In another embodiment, the bio-sourced glycolic acid composition does not comprise ethylene glycol and contains less than 15 ppm ammonium ions. Ethylene glycol and ammonium ions are two molecules described in the glycolic acid solution obtained according to the process described in EP1947079B1.

In a specific embodiment, the glycolic acid is present in a percentage of at least 65% w/w, preferably at least 70% w/w, in the composition of the invention.

In a specific embodiment of the invention, the final composition obtained is a liquid solution containing:

- bio-sourced glycolic acid dissolved in water at a ratio w/w of at least 65%, and preferably of at least 70%,

- succinic acid, in a concentration comprised between 500 and 5000 ppm, preferably between 1000 and 3000 ppm and even more preferably, between 1500 and 2500 ppm,

- uracil, in a concentration lower than 500 ppm, preferably lower than 200 pm, or lower than 100 ppm, most preferably comprised between 10 and 100 ppm,

- optionally, glyoxylic acid, in a concentration lower than 200 ppm, preferably lower than 150 ppm, and most preferably comprised between 5 and 150 ppm.

The present invention also concerns a composition as described above, obtained by the process as described above.

### *Uses of the composition of bio-sourced glycolic acid*

Glycolic acid has a broad spectrum of applications, including uses in leather, laundry and textile industry, in cleaning products, in polymers production, as an additive in plastics and as a food preservative

Glycolic acid is a member of the group of alpha hydroxy acids (AHAs) which have been used in cosmetic products for over 50 years. AHAs aid in the reduction of wrinkles as well as to soften strong, defining lines and improve the overall look and feel of the skin. They are also used as chemical peels. AHAs have an impact on keratinization, which is clinically detectable by the formation of a new stratum corneum. Further, glycolic acid, on topical application to photodamaged skin, has been shown to produce increased amounts of mucopolysaccharides and collagen and increased skin thickness without detectable inflammation. Cosmetic compositions comprising glycolic acid have been disclosed in multiple patent applications, for example in WO9709027, WO2016120796, WO2019133237 and WO2021226632.

As mentioned, the glycolic acid composition of the invention is bio-sourced. This is advantageous for cosmetic applications, since chemically produced glycolic acid compositions contain by-products having side-effects, such as skin irritation. In particular, chemical production processes of glycolic acid generate by-products such as formaldehyde and methoxyacetic acid, which are both CMR (Carcinogenic, Mutagenic and Reprotoxic) agents.

The composition of the invention may be used in any industry but is particularly adapted for dermatologic and cosmetic applications, as a skin care agent and/or as a peeling agent.

In an aspect, the invention concerns the use of a composition of bio-sourced glycolic acid as described above for cosmetic applications, in particular as a moisturizing agent or as an exfoliating agent.

The present invention also concerns cosmetic or dermatologic compositions comprising, as an active agent, a bio-sourced glycolic acid composition as described above.

These cosmetic or dermatologic compositions are adapted for a topical administration on skin and/or appendages (notably nails) and/or hair. They confer hydration of the superficial layers of the epidermis, limitation of water loss, limited irritant action and a peeling effect.

These cosmetic or dermatological compositions have a better skin tolerance than the commercially available, chemically produced glycolic acid.

Further, these cosmetic or dermatologic compositions induce a lower inflammatory response *in vitro,* and a better hydration of the superficial layers of the epidermis, than chemically produced glycolic acid.

Without wishing to be bound by any theory, the inventors think that this is due to a greater release of interleukin-1 alpha induced with a bio-sourced glycolic acid, in comparison with a chemically produced glycolic acid.

## EXAMPLES

The present invention is further defined in the following examples. It should be understood that these examples, while indicating preferred embodiments of the invention, are given by way of illustration only. The person skilled in the art will readily understand that these examples are not limitative and that various modifications, substitutions, omissions, and changes may be made without departing from the scope of the invention.

### I- Process of extraction and purification of glycolic acid

### Example 1: Recovery of clarified and purified glycolic acid solution from fermentation broth of *Escherichia coli* strain.

#### 1. Fermentation and pretreatment of the fermentation broth

*Escherichia coli* recombinant strain genetically modified as described in the patent application WO2018/138240, overexpressing the gene *xfp* from *Bifidobacterium animalis* (strain n°10), was fermented in 5m$^3$ fed-batch fermenter to produce glycolic acid.

Fermentation was performed on minimum medium with a feed of carbohydrates (such as glucose syrup) as carbon source. The temperature of the culture was maintained constant between 30°C and 34°C, preferentially 31°C and the pH was permanently adjusted to values between 6.0 and 7.5, preferentially 6.8 using an NH$_4$OH 28% solution.

Fermentation is stopped when glycolic acid (GA) titer is between 3.5% and 5% in the broth.

The recovered broth undergoes a desactivation/ pretreatment step which is a thermal treatment using heat exchangers and/or steam injection. Pretreatment conditions applied to glycolic acid fermentation broth (temperature and residence time) are sufficient to obtain a F0 value between 0.5 and 10, preferentially between 2 and 7, so that bacteria viability is lower than 0.1 CFU/mL in the pretreated broth.

The term F0 value designates the equivalent exposure time at 121.1°C to that of the actual exposure time at a variable temperature calculated with a temperature coefficient of the destruction of 10°C:

The **Table 1** below presents the evolution of the composition of two fermentation broths after pretreatment step performed on a continuous sterilization device.

**Table 1: Evolution of the composition of a fermentation broth after pretreatment step.**

| Sample reference | | AG28-AS100-RUN1 | AG28-SS100-RUN1 | AG28-AS100-RUN2 | AG28-SS100-RUN2 |
|---|---|---|---|---|---|
| Description | | Fermentation broth | Pretreated broth | Fermentation broth | Pretreated broth |
| Compound | Unit | | | | |
| Trehalose + phosphate | g/kg | 10.0 | 9.9 | 9.2 | 9.9 |

(continued)

| Sample reference | | AG28-AS100-RUN1 | AG28-SS100-RUN1 | AG28-AS100-RUN2 | AG28-SS100-RUN2 |
|---|---|---|---|---|---|
| Description | | Fermentation broth | Pretreated broth | Fermentation broth | Pretreated broth |
| Compound | Unit | | | | |
| Glucose | g/kg | 3.3 | 2.9 | 2.3 | 2.1 |
| Oxalate | g/kg | 1.2 | 1.3 | 1.3 | 1.3 |
| Orotate | g/kg | 0.9 | 0.9 | 0.9 | 0.9 |
| Glycolate (GA) | g/kg | 39.3 | 39.7 | 39.9 | 40.2 |
| Acetate | g/kg | 2.8 | 3.2 | 3.8 | 3.3 |
| TSC (total solid content) | % w/w | 5.7% | 5.3% | 5.7% | 5.5% |
| TSS (total suspended solid) | % w/w | 1.5% | 1.2% | 1.5% | 1.2% |

Pretreatment causes a slight decrease in total solid content due to Maillard Reaction (water forming reaction) and some decarboxylation reaction (carbon dioxide forming reaction). It also causes a decrease of total suspended solid content, due to solubilization of macromolecules from biomass.

A slight increase of glycolic acid is observed. A slight decrease of glucose is also observed, due to Maillard reactions. Main organic compounds titers are almost stable and the relative purity of glycolic acid in term of concentration ratio versus impurities has no variation above 10%, as shown in **Table 2.**

**Table 2: Comparison of concentration ratio of organic compound after pretreatment step.**

| Sample reference | AG28-AS100-RUN1 | AG28-SS100-RUN1 | AG28-AS100-RUN2 | AG28-SS100-RUN2 | Ratio variation (average of both runs) |
|---|---|---|---|---|---|
| Description | Fermentation broth | Pretreated broth | Fermentation broth | Pretreated broth | |
| Compound | | | | | |
| Trehalose + phosphate | 24.2% | 24.9% | 23.0% | 24.7% | 2.4% |
| Glucose | 7.2% | 6.2% | 5.9% | 5.2% | 9.3% |
| Oxalate | 3.3% | 3.2% | 3.4% | 3.1% | -1.8% |
| Orotate | 2.3% | 2.3% | 2.3% | 2.3% | -0.1% |
| Acetate | 8.3% | 8.2% | 9.5% | 8.3% | -7.1% |

2. Clarification of the fermentation broth

After pretreatment of the fermentation broth, cells and precipitated proteins are separated using a clarification step. Ultrafiltration (UF) has been used for clarification of the fermentation broth. In order to increase glycolic acid recovery yield, diafiltration have also been performed after filtration by addition of water in the filtration retentate. The **Table 3** presents the mass balance of an ultrafiltration run performed with a tangential filtration pilot using ultrafiltration ceramic membranes. Final concentration factor was 7.5 and global diafiltration ratio about 3.

Table 3: Results of ultrafiltration run AG27-S200-RUN2. *ND: Not Determined.*

| Sample reference | | AG27-AS200-RUN2 | Water | AG27-PS200-RUN2 | AG27-RS200-RUN2 | Mass balance (%) |
|---|---|---|---|---|---|---|
| Description | | Pretreated broth | Diafiltration water | Total Filtrate | Final Retentate | |
| Total mass (kg) | | 476.5 | 188.1 | 601.0 | 61.8 | 99.7% |
| Compound | Unit | | | | | |
| Trehalose + phosphate | g/kg | 9.4 | | 7.1 | 0.8 | 96.4% |
| Glucose | g/kg | 3.5 | | 2.6 | 0.3 | 96.6% |
| Oxalate | g/kg | 1.2 | | 0.9 | 0.1 | 97.3% |
| Orotate | g/kg | 0.8 | | 0.6 | 0.1 | 96.5% |
| Glycolate (GA) | g/kg | 40.4 | | 30.2 | 2.5 | 95.1% |
| Acetate | g/kg | 2.8 | | 2.1 | 0.1 | 94.4% |
| TSC (total solid content) | % w/w | 5.9% | | 3.3% | 12.0% | 96.4% |
| TSS (total suspended solid) | % w/w | 1.1% | | 0.0% | ND | ND |
| [Glycolate] / [TSC] | % | 68.0% | | 91.6% | 2.1% | |

Biomass elimination increased the relative purity of glycolic acid versus total dry matter (TSC) content. All the suspended solid is retained and concentrated in the retentate. The ratios between soluble organic and inorganic impurities and glycolic acid are slightly the same in filtrate and alimentation (pretreated broth). The ultrafiltration step is considered as a clarification step since it eliminates suspended solid only and it does not retain any soluble compound.

3. <u>Membrane nanofiltration step (step a)</u>

Ultrafiltration filtrate was then purified by a tangential filtration step using an organic nanofiltration (NF) membrane. Ultrafiltration permeate was concentrated up to 6 times (volume concentration factor of 6). Then, water is added in the retentate to perform diafiltration in order to enhance glycolic acid recovery yield. A transmembrane pressure between 20 and 30 bars is applied during the nanofiltration step, with an organic membrane having a cut-off of 300 Dalton. Results of a typical nanofiltration run are given in **Table 4.**

Table 4: **Results and mass balances of nanofiltration run AG28-S300-RUN1 (step a).** *UF: Ultrafiltration. NF: Nanofiltration. ND: Not Determined.*

| Sample reference | | AG28-AS300-RUN1 | Water | AG28-PS300-RUN1 | AG28-RS300-RUN1 | AG28-RUN2-RINSE | Mass balance (%) |
|---|---|---|---|---|---|---|---|
| Description | | UF Filtrate | Diafiltration water | Total NF Filtrate | Final NF Retentate | Skid rinsing water | |
| Total mass (kg) | | 1192.5 | 566.2 | 1483.0 | 190.0 | 83.6 | 99.9% |
| Compound | Unit | | | | | | |
| Trehalose | g/kg | 4.1 | | 0.1 | 26.1 | 3.5 | 106% |
| Glucose | g/kg | 2.2 | | 1.0 | 4.9 | 0.7 | 94.6% |
| Oxalate | g/kg | 1.1 | | 0.1 | 4.8 | 0.5 | 89.7% |
| Orotate | g/kg | 0.7 | | 0.6 | 0.1 | 0.0 | 94.1% |
| Glycolate | g/kg | 31.3 | | 23.9 | 1.6 | 0.9 | 96.0% |
| Acetate | g/kg | 2.9 | | 2.2 | 0.0 | 0.0 | 95.2% |

(continued)

| Sample reference | | AG28-AS300-RUN1 | Water | AG28-PS300-RUN1 | AG28-RS300-RUN1 | AG28-RUN2-RINSE | Mass balance (%) |
|---|---|---|---|---|---|---|---|
| Description | | UF Filtrate | Diafiltration water | Total NF Filtrate | Final NF Retentate | Skid rinsing water | |
| Total mass (kg) | | 1192.5 | 566.2 | 1483.0 | 190.0 | 83.6 | 99.9% |
| Compound | Unit | | | | | | |
| Uracil | g/kg | 0.1 | | 0.1 | 0.0 | 0.0 | 94.1% |
| Succinate | g/kg | 0.5 | | 0.2 | 1.2 | ND | 93.2% |
| Phosphate | g/kg | 0.5 | | 0.2 | 1.6 | ND | 90.8% |
| Ammonium | g/kg | 9.9 | | 6.4 | 8.2 | ND | 93.5% |

Nanofiltration step has a purification impact on soluble compounds, due to the Cut-off of the membrane. **Table 5** below presents the purification ratios for the main organic and inorganic impurities, between the ultrafiltration filtrate and the nanofiltration filtrate.

Table 5: Concentration ratio of soluble impurities versus glycolic acid after nanofiltration step (step a).

| Sample reference | AG28-AS300-RUN1 | AG28-PS300-RUN1 | |
|---|---|---|---|
| Description | Ultrafiltration Filtrate | Nanofiltration Filtrate | Purification ratio (%) |
| Compound | [Compound]/[GA] | [Compound]/[GA] | |
| Trehalose | 13.0% | 0.5% | 96.0% |
| Glucose | 7.1% | 4.3% | 39.6% |
| Oxalate | 3.4% | 0.5% | 84.5% |
| Orotate | 2.4% | 2.3% | 3.0% |
| Acetate | 9.3% | 9.4% | -0.2% |
| Uracil | 0.4% | 0.4% | 1.7% |
| Citrate | 2.7% | 0.6% | 79.5% |
| Succinate | 1.7% | 1.0% | 39.9% |
| Phosphate | 1.7% | 0.8% | 55.1% |
| Ammonium | 31.6% | 26.7% | 15.5% |

Nanofiltration step increased the relative purity of glycolic acid, as it retained sugars, di- or tri- organic acids (such as citrate, oxalate and succinate) and some inorganic anions (phosphate and sulphate - non presented in this example due to very low content in UF permeate). Nanofiltration had no purification efficiency for acetate (smaller molecule than glycolate), orotate and uracil (bigger molecules, but with a pyrimidinedione cycle that seems to help them to pass through the nanofiltration membrane).

**Example 2: Purification of nanofiltration filtrate containing glycolic acid using two ion exchange resins steps (step b).**

The pH of the nanofiltration permeate from step a is typically comprised between 5.5 and 6.5. As ammonia is used to regulate the pH during fermentation, glycolic acid - and the other organic acids - are mainly in ammonium salt form.

The NF permeate is then passed through a cation exchange resin bed (**step b1**) in order to bind the cations from the feeding solution and convert all organic acid in free acid form. The resin used for purification and acidification was a strong cationic resin from Purolite®. Two 210L resin beds were used in serial mode, and the volume of feeding solution engaged for one run of **step a** was the one of a run of nanofiltration step, so that saturation of the second resin bed was not over-passed. pH and conductivity are monitored at outlet of both resin bed to follow the absorption and identify when saturation was achieved (pH and conductivity increased at saturation). Once all the NF permeate was processed at a flowrate between 1 and 2 bed volume per h (210 to 420L/h), water was added to push the residual nanofiltration permeate in the two resin beds, in order to recover as much glycolic acid as possible. Recovery of treated product was stopped when conductivity decreased, meaning that all glycolic acid solutions have been correctly recovered. Resin beds were then regenerated by adding a mineral acid solution, to replace ammoniums by protons. The **Table 6** presents the results of a typical cation exchange resin run.

**Table 6**: Results of step b1 run AG28-R100-RUN1: elimination of ammoniums in nanofiltration permeate and impact on relative purity.

| Sample reference | AG28-PS300-RUN1 | | AG28-R100-PT-RUN1 | |
|---|---|---|---|---|
| Description | Total Permeate | | Total Treated Product | |
| pH | 6.03 | | 2.03 | |
| Compound | Titer (g/kg) | % vs [GA] | Titer (g/kg) | % vs [GA] |
| Trehalose | 0.2 | 0.6% | 0.1 | 0.3% |
| Glucose | 1.0 | 4.3% | 0.9 | 4.1% |
| Oxalate | 0.1 | 0.5% | 0.1 | 0.6% |
| Orotate | 0.6 | 2.3% | 0.5 | 2.3% |
| Glycolate (GA) | 23.9 | | 21.7 | |
| Acetate | 2.2 | 9.4% | 1.9 | 8.6% |
| Uracile | 0.1 | 0.4% | 0.1 | 0.4% |
| Phosphate | 0.2 | 0.8% | 0.1 | 0.7% |
| Ammonium | 6.4 | 26.7% | 0.02 | 0.09% |

Only ammoniums were adsorbed by the cationic resin. Other organic compounds remained almost in same ratio versus glycolic acid in the treated product of **step b1.** A slight dilution is observed. It is due to water recovery at beginning and end of glycolic acid solution recovery at outlet of the cationic resin bed, in order to limit glycolic acid losses.

The glycolic acid solution was then treated on a weak anionic resin in order to eliminate impurities whose pKa is lower than the one of glycolic acid (**step b2**). For this example, three 20 liters stainless steel columns were used in serial mode. The resin used was a weak anionic resin from Purolite®. Glycolic solution from step b1 was fed in weak anionic resin column at a flowrate of 3 bed volume per our (-60L/h). pH and conductivity were monitored at outlet of each column to identify the saturation of the resin, however the total resin volume was sufficient to eliminate the concerned impurities. The results of a typical weak anionic ion exchange resin run are given in the **Table 7 below.**

**Table 7**: Results of step b2 run AG25-R200-RUN1: elimination of stronger acid than glycolic acid in acidified nanofiltration permeate and impact on relative purity.

| Sample reference | AG25-R100-PT-RUN1 | | AG25-R200-PT-RUN1 | |
|---|---|---|---|---|
| Description | Treated Product on cationic resin | | Treated Product on anionic resin | |
| pH | 2.03 | | 2.15 | |
| Conductivity (mS/cm) | 3.37 | | 2.43 | |
| Compound | Titer (g/kg) | % vs [GA] | Titer (g/kg) | % vs [GA] |
| Trehalose | 0.3 | 1.3% | 0.2 | 0.8% |
| Glucose | 1.2 | 5.3% | 1.1 | 5.2% |
| Oxalic acid | 0.2 | 0.7% | 0.000 | 0.00% |
| Orotic acid | 0.4 | 1.9% | 0.009 | 0.04% |
| Glycolic acid (GA) | 22.1 | | 21.0 | |
| Formic acid | 0.1 | 0.5% | 0.1 | 0.4% |

(continued)

| Sample reference | AG25-R100-PT-RUN1 | | AG25-R200-PT-RUN1 | |
|---|---|---|---|---|
| Description | Treated Product on cationic resin | | Treated Product on anionic resin | |
| pH | 2.03 | | 2.15 | |
| Conductivity (mS/cm) | 3.37 | | 2.43 | |
| Compound | Titer (g/kg) | % vs [GA] | Titer (g/kg) | % vs [GA] |
| Acetic acid | 1.8 | 8.3% | 1.9 | 9.1% |
| Uracil | 0.1 | 0.3% | 0.1 | 0.3% |
| Succinic acid | 0.1 | 0.5% | 0.1 | 0.6% |
| Chloride | 0.022 | 0.1% | 0.002 | 0.01% |
| Phosphate | 0.151 | 0.7% | 0.000 | 0.00% |
| Sulphate | 0.004 | 0.02% | 0.001 | 0.00% |

The weak anionic ion exchange resin adsorbed oxalic acid, orotic acid and the inorganic anions. Weaker organic acids than glycolic acid (acetic, formic and succinic acids) and non-ionic compounds (sugars and uracil) were not adsorbed, and their concentration ratios versus glycolic acid remained stable.

The purified glycolic acid solution recovered after these two ion exchange resin steps was then concentrated and further purified in Example 3.

**Example 3: Concentration of glycolic acid using two evaporation steps (steps c1 and c3) and a nanofiltration step (step c2)**

The purified glycolic acid solution from **step b2** needs to be concentrated for further purification steps. For the first preconcentration step by evaporation (**step c1**), two fluxes from further steps are recycled with the purified glycolic acid solution, due to their glycolic acid contents. The first one is the diafiltration permeate of nanofiltration step (**step c2**) and the second one is the mother liquor of the crystallization step (**step d**). These recycling loops are described in the **Figure 1.**

The first evaporation of **step c** was performed using a distillation column with a double jacket stirred tank for heat transfer. Steam was supplied in the double jacket and water was evaporated under vacuum (400 mbarA) until glycolic concentration in the concentrated solution was between 25 and 35% w/w. This step was started in batch mode, and then operated in continuous mode in order to concentrate the whole feeding solution prepared for the run. Diluted glycolic acid solution was fed in the stirred tank. Distillates were condensed using a heat exchanger supplied be cooling water. Results of the typical **step c1** run AG28-B130-RUN1 are presented in **Table 8.**

| Sample reference | AG27-S400-RUN1-PDNFA | AG27-CR-RUN1-ML | AG28-R200-PT | AG28-TB130-RUN1 | AG28-PB130-RUN1 | Mass balance (%) |
|---|---|---|---|---|---|---|
| Description | Diafiltrate of previous nanofiltration run (step c2) | Mother liquor of previous crystallization run (step d) | Diluted GA solution from step b2 | Distillate | Preconcentrated GA solution | |
| Mass (kg) | 92.8 | 89.8 | 2723.9 | 2502.5 | 319.8 | 97.1% |
| Compound | g/kg | g/kg | g/kg | g/kg | g/kg | |
| Glucose | 0.3 | 1.9 | 0.9 | 0.0 | 6.4 | 78% |
| Glyoxylic acid | 0.2 | 1.0 | < LOQ | 0.0 | 0.4 | 106% |
| Glycolide (GA) | 0.0 | 119.7 | 0.0 | 0.0 | 27.0 | 80% |
| Glycolic acid (GA) | 109.1 | 406.0 | 20.4 | 0.1 | 336.3 | 105% |
| Formic acid | 0.2 | 1.0 | 0.1 | 0.1 | 0.2 | 92% |
| Acetic acid | 0.6 | 2.0 | 1.9 | 1.7 | 1.8 | 93% |
| Uracil | 1.7 | 5.0 | 0.1 | 0.0 | 2.6 | 99% |
| Succinic acid | 2.8 | ND | 0.1 | 0.0 | 6.2 | ND |

Table 8: Results of step c1 run AG28-B130-RUN1: partial elimination of light impurities and preconcentration of glycolic acid. *ND: Not Determined. LQQ: Limit Of Quantification of HPLC system.*

Some Maillard reactions consumed a part of the glucose, so that glucose mass balance was under 80%. Glycolic acid mass balance was 5% above 100% due to glycolide hydrolysis. Glycolide from crystallization mother liquors is naturally hydrolysed in 2 glycolic acid molecules when glycolic acid titer is below 20%, due to equilibrium constant between the 2 forms of glycolic acid. Glycolide mass balance was under 80% due to this equilibrium.

Lightest organic acids were recovered in distillates: 82% of acetic acid and 74% of formic acid. Concentration of these two light impurities remained slightly the same in feeding solution and distillate of evaporation **step c1.**

Table 9: **Impact of evaporation step c1 on purity of glycolic acid.** *ND: Not Determined. LOQ; Limit Of Quantification of HPLC system.*

| Sample reference | AG28-R200-PT-POOL | | AG28-PB130-RUN1 | |
|---|---|---|---|---|
| Description | Diluted GA solution from step b2 (RUN1+RUN2) | | Preconcentrated GA solution | |
| Compound | Titer (g/kg) | % vs [GA] | Titer (g/kg) | % vs [GA] |
| Glucose | 0.9 | 4.3% | 6.4 | 1.9% |
| Orotic acid | 0.0 | 0.0% | 0.1 | 0.0% |
| Glyoxylic acid | <LOQ | ND | 0.3 | 0.1% |
| Glycolide (GA) | 0.0 | 0.0% | 27.0 | 8.0% |
| Glycolic acid (GA) | 20.4 | | 336.3 | |
| Formic acid | 0.1 | 0.6% | 0.2 | 0.1% |
| Acetic acid | 1.9 | 9.1% | 1.8 | 0.5% |
| Uracil | 0.1 | 0.4% | 2.6 | 0.8% |

(continued)

| Sample reference | AG28-R200-PT-POOL | | AG28-PB130-RUN1 | |
|---|---|---|---|---|
| Description | Diluted GA solution from step b2 (RUN1+RUN2) | | Preconcentrated GA solution | |
| Compound | Titer (g/kg) | % vs [GA] | Titer (g/kg) | % vs [GA] |
| Succinic acid | 0.1 | 0.7% | 6.2 | 1.8% |

Evaporation **step c1** has a positive impact on formic and acetic acid concentration ratio versus glycolic acid. Recycling the diafiltrate of **step c2** and the mother liquor of crystallization **step d** has also an impact on relative purities:

- succinic acid and glyoxylic acid ratios increased due to succinic acid and glyoxylic acid recycling from mother liquors, however a small part of glyoxylic acid should come from the diluted glycolic acid solution from **step b2** (<LOQ: less than 5ppm)
- glucose ratio decreased due to significantly lower glucose ratio in recycled mother liquors and diafiltrate.

Concentrated glycolic acid solution was then purified by a tangential filtration step using an organic nanofiltration membrane (**step c2**). Due to the low pH of the feeding solution, acidic organic membranes are preferably used for this step, considered as a nanofiltration of acid (NFA). 30% GA solution was concentrated up to 8 times (volume concentration factor of 8). Then, water is added in the retentate to perform diafiltration in order to recover glycolic acid from the retentate and recycle this diafiltrate in evaporation **step c1** feeding solution. A transmembrane pressure between 20 and 30 bars is applied during the NFA step, and the organic membrane has a mininum MgSO4 rejection of 98%. Results of a typical **step c2** nanofiltration run are given in **Table 10**.

| Sample reference | AG28-PB130-POOL | Water | AG28-PS400-RUN1 | AG28-S400-RUN1-PDNFA | AG28-RS400-RUN1 | Mass balance (%) |
|---|---|---|---|---|---|---|
| Description | Preconcentrated GA solution | Diafiltration water | Filtrate | Diafiltrate | Final Retentate | |
| Mass (kg) | 354 | 93.2 | 309.2 | 114.4 | 47.8 | 105.4% |
| Compound | g/kg | g/kg | g/kg | g/kg | g/kg | |
| Trehalose | 0.4 | | 0.0 | 0.0 | 2.9 | 104% |
| Glucose | 6.7 | | 0.4 | 0.9 | 40.4 | 92% |
| Orotic acid | 0.06 | | 0.04 | 0.04 | 0.07 | 95% |
| Glyoxylic acid | 0.4 | | 0.3 | 0.1 | 0.1 | 77% |
| Glycolide (GA) | 38.0 | | 13.9 | 20.9 | 40.9 | 64% |
| Glycolic acid (GA) | 353.6 | | 333.8 | 120.1 | 35.6 | 95% |
| Formic acid | 0.2 | | 0.2 | 0.1 | 0.3 | 90% |
| Acetic acid | 2.4 | | 2.3 | 0.5 | 0.4 | 90% |
| Uracil | 2.6 | | 2.1 | 1.5 | 0.7 | 94% |
| Succinic acid | 6.2 | | 5.2 | 2.7 | 3.5 | 95% |

Table 10: Results and mass balances of nanofiltration run AG28-S400-RUN1 (step c2).

About 83% of engaged glycolic acid in nanofiltration **step c2** were recovered in the filtrate of the preconcentrated solution. This step of nanofiltration increased glycolic acid purity due to elimination of residual trehalose and glucose.

Table 11: Concentration ratio of soluble impurities versus glycolic acid after nanofiltration step on concentrated glycolic acid solution (step c2).

| Sample reference | AG28-PB130-POOL | | AG28-PS400-RUN1 | |
|---|---|---|---|---|
| Description | Preconcentrated GA solution | | Filtrate | |
| Compound | Titer (g/kg) | % vs [GA] | Titer (g/kg) | % vs [GA] |
| Trehalose | 0.4 | 0.1% | 0.0 | 0.0% |
| Glucose | 6.7 | 1.9% | 0.4 | 0.1% |
| Glyoxylic acid | 0.4 | 0.1% | 0.3 | 0.1% |
| Glycolide (GA) | 38.0 | 10.7% | 13.9 | 4.2% |
| Glycolic acid (GA) | 353.6 | | 333.8 | |
| Formic acid | 0.2 | 0.07% | 0.2 | 0.05% |
| Acetic acid | 2.8 | 0.8% | 2.2 | 0.7% |
| Uracil | 2.6 | 0.7% | 2.1 | 0.6% |
| Succinic acid | 6.2 | 1.7% | 5.2 | 1.6% |

The nanofiltration on preconcentrated glycolic acid solution (**step c2**) eliminated the part of sugars that the first nanofiltration step (**step a**) had not retained. The preconcentrated glycolic acid solution was then almost free of sugars, what reduced the risk of Maillard reaction for the next evaporation step.

The second evaporation step (**step c3**) was performed under vacuum (550 mbarA) on the same equipment as the first evaporation (step c1), but with different operating conditions. Nanofiltration filtrate from **step c2** was fed in top of rectification column and water was fed in the double jacket stirred tank. at the beginning of the run, water was filled in the double jacket stirred tank. Once evaporation had begun, feeding of NFA filtrate and stripping water were started. A higher distillate flowrate than stripping water flowrate was applied by regulation of the steam injection in the double jacket, while level in the heated stirred tank remained stable, until total GA concentration was above 72% w/w. Once above 72% w/w of glycolic acid in the concentrated solution, the second evaporation **step c3** was operated in continuous mode. Distillates were condensed using a heat exchanger supplied be cooling water. Results of the typical **step c3** run AG28-B170-RUN1 are presented in **Table 12.**

| Sample reference | AG28-PS400-RUN1 | Water | AG28-TB170-RUN1 | AG28-PB170-RUN1 | Mass balance (%) |
|---|---|---|---|---|---|
| Description | Filtrate from step c2 | Total added water | Distillate | Concentrated GA solution | |
| Mass (kg) | 310.6 | 350.0 | 507.6 | 145.0 | 99% |
| Compound | g/kg | g/kg | g/kg | g/kg | |
| Glucose | 0.4 | | 0.0 | 0.5 | 51% |
| Orotic acid | 0.04 | | 0.00 | 0.1 | 96% |
| Glyoxylic acid | 0.3 | | 0.0 | 0.5 | 90% |
| Glycolide (GA) | 13.9 | | 0.0 | 111.7 | 375% |
| Glycolic acid (GA) | 333.8 | | 0.7 | 598.5 | 84% |
| Total GA | 352.0 | | 0.7 | 744.8 | 99% |
| Formic acid | 0.2 | | 0.2 | 0.0 | 173% |
| Acetic acid | 2.3 | | 1.6 | 0.08 | 121% |
| Uracil | 2.1 | | 0.0 | 4.5 | 101% |
| Succinic acid | 5.2 | | 0.0 | 11.3 | 101% |

Table 12: Results of stripping and evaporation run AG28-B1700-RUN1 (step c3).

The concentration of glycolic acid during evaporation **step c3** is much higher than 30% w/w. It has an impact on the glycolide-glycolic acid equilibrium, leading to glycolide formation from the glycolic acid. It is also enhanced by the water elimination in distillate. Then, mass balance for glycolic acid has to be calculated as "total glycolic acid", and glycolide concentration is converted in glycolic acid concentration after multiplication with 1.31 ratio (molar weight ratio between two glycolic acid molecules and one glycolide molecule). Mass balance of glucose was the only one under 95%, due to Maillard reactions during evaporation (nanofiltration filtrate was almost clear, but concentrated solution was yellowish). Light impurities mass balances were above 100%, probably due to underestimation in the nanofiltration filtrate, however, stripping eliminated more than 98% of acetic and formic acid in the final concentrated glycolic acid solution of **step c3.**

**Example 4: Crystallisation and recovery of purified glycolic acid crystals from concentrated glycolic acid solution (step d).**

Concentrated glycolic acid solution from step c was cooled in a 20L stainless-steel double jacket stirred tank (crystallizer) from 20°C to -15°C. Glycolic acid started to crystallize at 3°C after a seeding with 1g of GA crystals from a previous run. In previous crystallization runs, nucleation occurred between 8°C and 2°C with or without seeding. When temperature of the slurry was at -15°C, cooling was regulated to maintain temperature at -15°C during at least 1 hour. From the 19kg of glycolic acid slurry obtained, 10kg were discharged and treated in a vertical axis basket centrifuge (Robatel pilot centrifuge with 320mm basket and adapted removal filtration bag with a cut-off of approximately 50μm) to separate crystals and mother liquor. The centrifugation was operated between 400 and 1400g with a same efficiency in terms of residual moisture in the wet cake of crystals. An acceleration of 1300g was applied for each discharge of the crystallizer. A washing nozzle (supplied by Lechler) was used for the wash of the crystals with softened water. A minimum ratio of 3 % w/w of water was engaged for each discharge. A wash ratio of 5% w/w (kg water / kg of slurry) was applied. After each discharge, and until concentrated glycolic acid solution was completely treated, 10kg of fresh concentrated GA solution were added in the crystallizer. The diluted slurry obtained was cooled down to -15°C again, and after one hour at this temperature, a new discharge was performed.

The whole concentrated glycolic acid from **step c** was treated with these operating conditions. Results of a typical crystallization step (**step d**) is presented in **Table 13** below.

| Sample reference | AG28-PB170-RUN1 | Water | AG28-CR-CR | AG28-CR-ML+W+R | Mass balance in ML (%) |
|---|---|---|---|---|---|
| Description | Concentrated GA solution | Total added water | GA crystals | Mother liquor + washing + rinsing | |
| Mass (kg) | 145.0 | 14.5 | 32.2 | 127.6 | 100% |
| Compound | g/kg | g/kg | g/kg | g/kg | |
| Glucose | 0.5 | | ND | 1.0 | 188% |
| Orotic acid | 0.09 | | ND | 0.1 | 88% |
| Glyoxylic acid | 0.54 | | 0.01** | 0.64 | 104% |
| Glycolide (GA) | 111.7 | | ND | 99.1 | 78% |
| Glycolic acid (GA) | 598.5 | | 958.7* | 477.2 | 70% |
| Total GA | 744.8 | | ND | 607.0 | 72% |
| Uracil | 4.5 | | 0.01** | 4.94 | 97% |
| Succinic acid | 11.3 | | 3.3** | 11.9 | 93% |

<u>Table 13:</u> Results of crystallization run AG28-CR (lab reference AG24039CR) (step d)

*: total dry matter of glycolic acid crystals is considered as pure glycolic acid (almost > 99% pure).

**: succinic acid, glyoxylic acid and uracil concentrations have been calculated from concentrations measured in the further solution of dissolved crystals (step e). Other impurities were mainly recovered in mother liquor, and their concentrations were under the limit of quantification of HPLC system (LOQ) in the crystals solution (step e).

*ND: Not Determined.*

At the end of **step d,** about 28% of total glycolic acid were recovered in crystals. Mother liquors were then recycled in evaporation **step c1,** so that glycolide can be hydrolysed in two glycolic acid molecules. Crystallisation is very specific to glycolic acid, and only a small amount of succinic acid co-crystallised with GA. Other impurities were eliminated by the crystallisation **step d.**

As glycolic acid is commercialized as a 70% w/w solution for cosmetics, crystals were resolubilized in a further step.

**Example 5: Dissolution and polishing of purified glycolic acid crystals (step e).**

3.3kg of softened water was filled in a double jacket stainless-steel reactor. 9.4kg of crystals from AG24039CR run (discharge references: 3, 4, 5, 7 and 8) were added in the reactor with heating (<80°C), until all crystals were solubilized (step e1).

The APHA/Platinum-Cobalt color scale is described in ASTM D1209 "Standard Test Method for Color of Clear Liquids (Platinum-Cobalt Scale)", and a colorimeter Lovibond was used. APHA/Pt-Co color of the purified glycolic solution recovered after complete dissolution of crystals from **step d** had a value of 27 mg/L Pt-Co.

Dissolution **step e1** was completed with a polishing step (**step e2**) by passing through an activated carbon. Different sources of activated carbon had been validated for color removal: coal, coconut and wood. Two references of granulated activated coal were used:

- a bituminous coal activated carbon: BAC.
- a coconut activated carbon: CAC.

The **Table 14** presents the results of the polishing performed for each carbon reference on a 300mL glass column filled by 200mL of activated carbon.

**Table 14: Color and composition of purified glycolic acid solution at end of step e. *ND: Not Determined.***

| Sample reference | | AG24043MX | AG24043CC1-PT | AG24043CC2-PT |
|---|---|---|---|---|
| Description | | Purified GA solution after GA crystals dissolution (step e1) | GA solution polished on CAC (step e2) | GA solution polished on BAC (step e2) |
| Mass (kg) | | 11.4 | 5.3 | 5.1 |
| Yield vs engaged GA (%) | | | 90% | 91% |
| APHA color (mg/L Pt-Co) | | 26.6 | 2.6 | 1.3 |
| Compound | Unit | | | |
| Total GA | % w/w | 71.5 | 71.4 | 71.3 |
| Glyoxylic Acid | ppm | ND | < 50 (-10) | < 50 (~11) |
| Uracil | ppm | ND | 11 | 5 |
| Succinic Acid | ppm | ND | 2522 | 2513 |
| Formic Acid | ppm | ND | 0 | 0 |
| Acetic Acid | ppm | ND | 0 | 0 |
| Ammonium ions | ppm | ND | 3 | 2 |
| Diglycolic Acid | ppm | ND | 0 | 0 |
| Methoxyacetic Acid | ppm | ND | 0 | 0 |
| Formaldehyde | ppm | ND | <5 | <5 |
| Malic Acid | ppm | ND | 0 | 0 |

APHA color was sharply decreased with both activated carbon, and was lower than the standard APHA color value for cosmetic glycolic acid < 15 mg/L Pt-Co.

Concerning impurities, succinic acid was the main impurity of the purified glycolic acid solution obtained at the end of **step e.** The other identified impurities were glyoxylic acid and uracil, but at very low concentration for these runs. Limit of quantification (LOQ) of glyoxylic acid is considered at 50ppm, the concentrations given between parentheses are indicative ones.

Light impurities that are produced during the fermentation step - acetic acid and formic acid - were not detected in final product by HPLC.

The four last compounds have never been identified in fermentation broth, but analysis on final product confirmed that they did not appear during the purification steps. These compounds are typical impurities of chemically produced glycolic acid.

**Example 6: Comparison of final glycolic acid solution batches produced at two different scales, according to the process as described in example 1 to 5.**

During process development, different batches of final product were produced following the different steps described in this invention. Some parameters have been optimized during the development, and the main parameter changes are described in the **Table 15.** The table below also presents main impurities concentration at end of **step e.**

**Table 15**: Color and composition of purified glycolic acid solution batches produced following the steps described in this invention.

| Batch reference | | GAB0001 | GAC0001 | GAC0002 | GAC0003 | GAD0001 | GAD0002 |
|---|---|---|---|---|---|---|---|
| Modified step | | typical | step d + step e | | | Step d + step e | |
| Process variation | | | step d: acceleration reduced to 400g<br>step e: different activated carbon | | | activated carbon | |
| Analytical result | unit | | | | | | |
| Color | mg/L Pt-Co | 2 | 1 | 1 | 1 | 3 | 2 |
| Total Glycolic Acid (GA) | % w/w | 70,2 | 71,0 | 71,5 | 71,1 | 71,4 | 71,3 |
| Uracil | ppm | 33 | 7 | 33 | 40 | 11 | 5 |
| Succinic Acid | ppm | 2635 | 2648 | 2391 | 2380 | 2522 | 2513 |
| Glyoxylic acid | ppm | 14 | 52 | 19 | 19 | 11 | 10 |
| Ammonium ions | ppm | 8 | 11 | 9 | 5 | 3 | 2 |

The purified glycolic acid composition obtained with the claimed process, with or without further purification steps, presents the following features:

- Uracil and glyoxylic acid concentration are lower than 100 ppm;
- Succinic acid concentration is stable between 2000 and 3000 ppm, meaning that co-crystallization of succinic acid with glycolic acid is stable and reproducible.

**II- Application in cosmetics**

In the above examples, two distinct glycolic acids (GA) were tested and compared with respect to their impact on tissue viability, inflammatory response and hydration of the surface layers of the skin. **Table 16** describes both glycolic acids used in the following examples.

**Table 16: Description of both tested glycolic acids.**

| GA-METEX (Glycolic Acid *according to the invention*) | Bio-sourced glycolic acid obtained by the process of the present invention from fermentation of *Escherichia coli* strain |
|---|---|
| **GA-CONTROL** | Commercially available, chemically produced glycolic acid |

**Example 7: Measurement of tissue viability and inflammatory response on a 3-dimensional reconstituted human epidermis model.**

To assess the skin tolerance of glycolic acid solution of the invention, and to compare it with a common chemically produced glycolic acid. The *in vitro* experimental approach is based on the measurement of tissue viability and inflammatory response, performed on a 3-dimensional (3D) reconstituted human epidermis model after topical application of the tested compounds.

1. Protocol

*Test system and test procedure:*

The 3D reconstituted human epidermis (RhE) used as test system in the study, is the SKINETHIC™ RHE model commercially available from the company EPISKIN SA. The SKINETHIC™ RHE model is obtained by tissue engineering from untransformed human keratinocytes previously obtained from human epidermis. The model is described in the OECD 439 guideline for *in vitro* skin irritation of chemical products.

The assay is based on topical application of the tested GA at different concentrations on reconstructed human epidermis (RHE SkinEthic™), followed by evaluation of:

- Tissue viability, by MTT colorimetric test to determine the concentration of test product needed to produce the viability to 50% of control tissues ($EC_{50}$).
- SkinEthic™ RHE tissue inflammatory response, by interleukin 1α (IL-1α) release assay.

The assay takes place in two steps:

- Step 1: $EC_{50}$ concentration finding assay is performed with one representative sample.
- Step 2: determination of $EC_{50}$ concentration values and IL-1α levels performed with both GA.

The SKINETHIC™ RHE epidermal tissues are transferred into 6-wellculture plates previously filled with maintenance medium 5epiSkin™ followed by incubation under standard conditions:

- Temperature 37°C ($\pm 1$ °C).
- Humidified 5% ($\pm 1$°C) $CO_2$ atmosphere,

to synchronize the tissue age with the testing schedule, prior to experiment.
Control group: RHE tissues untreated.
Treated group: RHE tissues treated with GA.

Prior to GA application, tissue inserts are transferred in 12-well plates prefilled with fresh maintenance medium. 60 μl/cm² of each GA solution is applied on top of epidermidis. After application, tissues are incubated for 24 hours at 37°C ($\pm 1$ °C) and 5% ($\pm 1$°C) $CO_2$.

### Tissue viability - MTT assay

The tissue viability is measured with the MTT assay performed in 24 hours. The MTT assay is based on the reduction of 3-(4,5-Dimethylthiazol-2-yl)-2,5-Diphenyltetrazolium Bromide (MTT) into insoluble purple formazan crystals, by metabolically active cells in a mitochondria-dependant reaction catalysed by succinate dehydrogenase. The ability of cells to reduce MTT after exposure to GA, compared to the control (untreated epidermis), enables to deduce the relative toxicity of the applied GA. The optical density (OD) of formazan solution is proportional to the number of viable cells.

### MTT assay:

After incubation, tissues are rinsed with PBS and then transferred into 24-well plates pre-filled with MTT solution. After 120 minutes ($\pm 10$ minutes) of incubation at 37°C ($\pm 1$ °C), the tissues are transferred into 24-well plates containing DMSO for extraction of formazan. Then the plates are stored overnight at room temperature. At the end of the extraction period, 3 x 200 μl aliquots of formazan extracts are transferred into a 96-well flat bottom microtiter plate and the optical density is read at 570 nm in a microplate reader.

### Test viability expression

The optical densities of each replicate are corrected by substracting the mean OD value of blank measured in parallel. The mean from corrected OD values ($OD_{corr}$) as well as standard deviation are calculated in each experimental group.

From the mean $OD_{corr}$ values, the relative tissue viability (% viab) is calculated as a percentage of the mean viability of the control (untreated epidermis):

$$\% \text{ viab (treated)} = \frac{(\text{treated}) \, ODcorr \, mean}{(\text{Control } ODcorr \, mean)} \times 100.$$

### Determination of $EC_{50}$ value

The graph represents the dose response curve of cells viability (% viab) versus the GA concentration (C). The

$EC_{50}$ value is calculated from the dose response curve, by using the probit-log regression model.

*IL-1α assay:*

At the end of the assay after 24 hours incubation period, the conditioned media underneath tissues are collected, aliquoted and stored at -20°C until IL-1α assay.

IL-1α is quantified using an Elisa kit (Quantikine Human IL-1α DLA50) according to the manufacturer's instructions. The optical densities are read at 450 nm.

2. Results

**Test viability expression**

The results are shown in **Table 17.** A dose dependent cytotoxicity is observed in the range of tested concentrations for the GA-METEX. The $EC_{50}$ value is 1.70%. In the case of the GA-control, a dose dependent cytotoxicity is also observed in the range of tested concentrations. The $EC_{50}$ value is 1.42%.

Table 17 **Tissue viability function of different tested concentration of glycolic acids.**

| Concentrations | GA-METEX | GA-CONTROL |
|---|---|---|
| **1.27%** | 74.9 | 62.1 |
| **1.69%** | 59,6 | 31.2 |
| **2.25%** | 22.2 | 10.5 |
| **3%** | 3.5 | 3.4 |
| **$EC_{50}$ (% p/v)** | 1.70 | 1.42 |

These results demonstrate that the GA-METEX is less cytotoxic than the chemically produced GA-CONTROL.

**Inflammatory response**

IL-1α levels (pg/ml) are interpolated from the standard curve built with IL-1α standard provided with the Elisa kit. The mean of IL-1α release concentrations is calculated in each experimental group. For the comparative analysis, the IL-1α levels were normalized in function of tissue viability (MMT test). The values R (IL-1α/Viab%) $\times 100$) have been calculated at different concentrations with the two GA and collected in the following **Table 18:**

Table 18: **IL-1α ratios.**

| R | GA-METEX | GA-CONTROL |
|---|---|---|
| **1.27%** | 10.42 | 15.09 |
| **1.69%** | 18.26 | 35.33 |
| **2.25%** | 35.56 | 75.07 |

The analysis of IL-1α ratios indicates that the two glycolic acids induce the release of IL-1α in a dose-dependent manner.

In the presence of GA-CONTROL, the IL-1α release quantity is almost the double of the IL-1α release obtained with GA-METEX, therefore GA-CONTROL application generates a stronger inflammation.

3. Conclusion

GA-METEX induces a lower inflammatory response *in vitro* when compared to GA-CONTROL.

**Example 8: Product safety and formulation**

The risk of skin irritation has been tested *in vivo.* Different formulas (required for *in vivo* studies) were tested comparatively:

- Cream with 5% GA-METEX
- Cream with 5% GA-CONTROL
- Cure with 10% GA-METEX
- Cure with 10% GA-CONTROL

"Cream" and "Cure" are identical in GA content and nature, but differ for ingredients and proportion in water.

1. Protocol

The Patch test (OECD 439) is used to determine the skin irritation potential of a product. The product is applied to the scapular area of the volunteers' backs. An empty cup serves as a control. 48 hours after application, the cups are removed and their location marked out with a skin pencil. Any skin reactions are taken 30 minutes after cup removal.

2. Results

Results are shown in **Table 19:**

**Table 19: Results of different formulations assays.**

| Formulation | Results |
| --- | --- |
| Cream 5% GA-METEX | Non-irritant |
| Cream 5% GA-CONTROL | Mild irritating |
| Cure 10% GA-METEX | Non-irritant |
| Cure 10% GA-CONTROL | Non-irritant |

3. Conclusion

GA-METEX does not show any irritant effect in cosmetic formulations and can be used without risk up to at least 10%.

**Example 9: Skin homeostasis**

As the skin ages, cell renewal slows down. Over time the renewal of the *stratum corneum* will lengthen and may take up to 80 days (instead of 28 days in young age). This will lead to modifications in the barrier function of the skin, leading to an increase of the imperceptible loss of water (TransEpidermal Water Loss, TEWL). This process results in a reduction in hydration and sebum secretion.

The instrumental test made it possible to assess the effects of the 2 glycolic acids on hydration, TELW and sebum parameters of the skin.

1. Protocol

A panel of 20 women, users of anti-aging cream, was selected based on age criterion (premature and mature skin) and skin hydration (normal to dry skin). Two creams containing respectively 5% of GA-METEX and 5% of GA-CONTROL, are applied daily to each half of the face by the panelistsfor 14 days. At 10 minutes after the first application (T10 minutes) and on the 14th day, instrumental measurements are carried out to measure the following parameters:

- Hydration of the superficial layers of the epidermis. The principle is based on the electrical conductivity of the skin. There is a direct relationship between the water content of the surface layers of the skin and its electrical capacity (capacitance). This measurement is carried out using a Corneometer®.
- TransEpidermal Water Loss (TEWL). TEWL corresponds to the passive diffusion of water through the *stratum corneum.* This measurement makes it possible to evaluate the integrity of the barrier function. When the barrier is altered, an increase in water flow towards the external environment is observed. The positive influence of a product on skin hydration will result in a reduction in TEWL. This measurement is carried out using a Tewameter®.
- Sebum secretion. The sebum present on the surface of the epidermis helps to maintain skin hydration. However, when production is excessive, it can give a shiny (oily) appearance and above all promote the formation of skin imperfections. Conversely, a lack of sebum will disrupt skin homeostasis and cause a dry skin. The measurement is carried out using a specific probe applied to the skin and fitted with a transparent film sensitive to sebum which allows its quantification.

2. Results

*Hydration of the superficial layers of the epidermis*

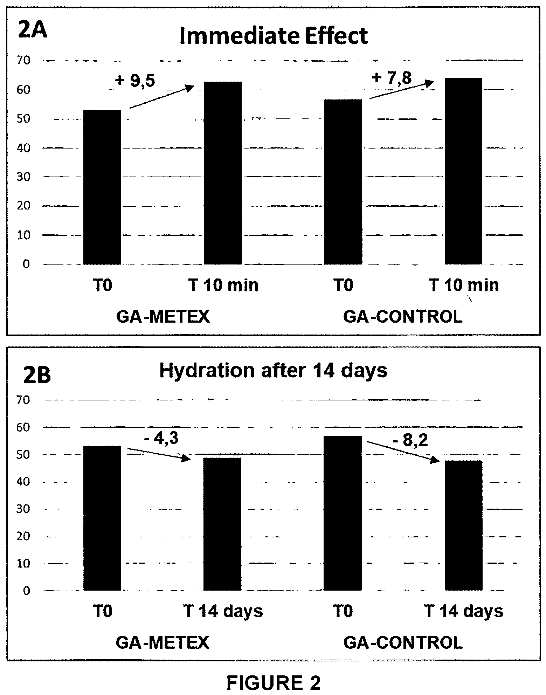

10 minutes after application of GA-METEX cream, the hydration state of the consumers skin is significantly higher than those measured at starting point, as shown in **Figure 2A** (with an increase rate of 18%). An increase in skin hydration is also observed with GA-CONTROL containing cream, but in a smaller proportion (with an increase rate of 13%).

After 14 days, the hydration state of the consumers skin decreases, as shown in **Figure 2B,** about 8% in the case of treatment with the GA-METEX cream and around 18% in the case of treatment with GA-CONTROL cream.

These results show that GA-METEX containing cream maintains a better hydration of the superficial layers of the epidermis than a cream containing chemically produced glycolic acid.

*TransEpidermal Water Loss (TEWL)*

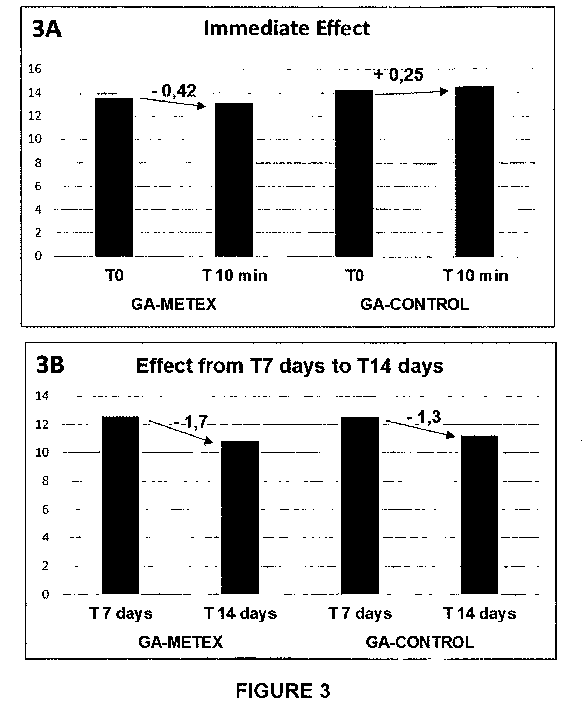

As soon as the two formulas are applied (10 min), a difference in behavior is noticed. In presence of GA-METEX cream, a reduction in TEWL is observed whereas in the case of GA-CONTROL cream, an increase in TEWL is observed. (**Figure 3A**).

Between the 7th day and the 14th day, a decrease in TEWL is observed for both creams GA-METEX and GA-CONTROL (**Figure 3B**). However, the decrease in TEWL when the GA-CONTROL cream is used is less important than the decrease when the GA-METEX is used. These results show that GA-METEX cream is more performant in terms of *TransEpidermal Water Loss* than the GA-CONTROL cream.

*Sebum secretion*

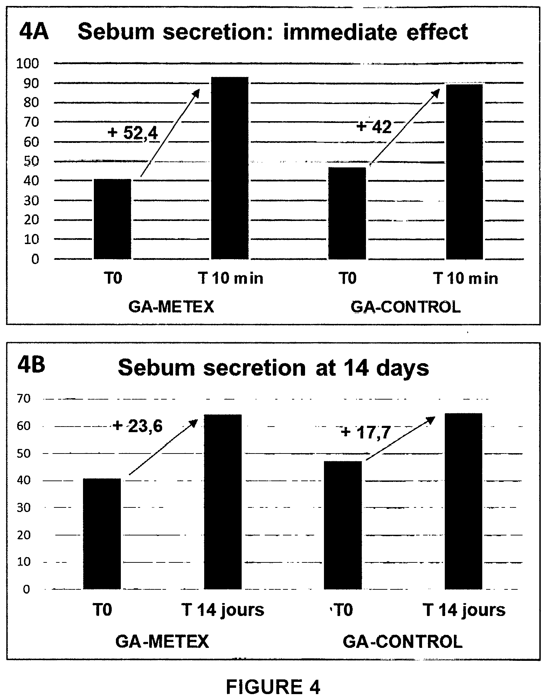

10 minutes after application of both creams, a significant increase of the quantity of sebum is observed (**Figure 4A**). An increase of about 128% is observed for the GA-METEX cream and of about 89,2% for theGA-CONTROL cream. After 14 days, the quantity of sebum increases by 60% for GA-METEX and by 37,5% for GA-CONTROL cream (**Figure 4B**).

Overall, these results show that sebum secretion is more important when the GA-METEX cream is used compared to the GA-CONTROL cream.

3. Conclusions

Overall, the GA-METEX cream induces a better hydration of the superficial layers of the epidermis and reduces the TEWL without altering the barrier function. Thus, the GA-METEX can be used in a cream, i.e., a dermatologic composition, for cosmetic application as a moisturizing agent.

**Example 10: Usage test**

1. Protocol

A panel of 60 women with mature skin ranging from normal to dry, were selected to apply to each half of the face, the two tested items: GA-METEX and GA-CONTROL. The panelists were asked about their feeling after 7 and 14 days of daily application. The questionnaire covered items: nourished skin, uniform skin, smooth skin, luminous skin, finer skin texture, firm skin.

2. Results

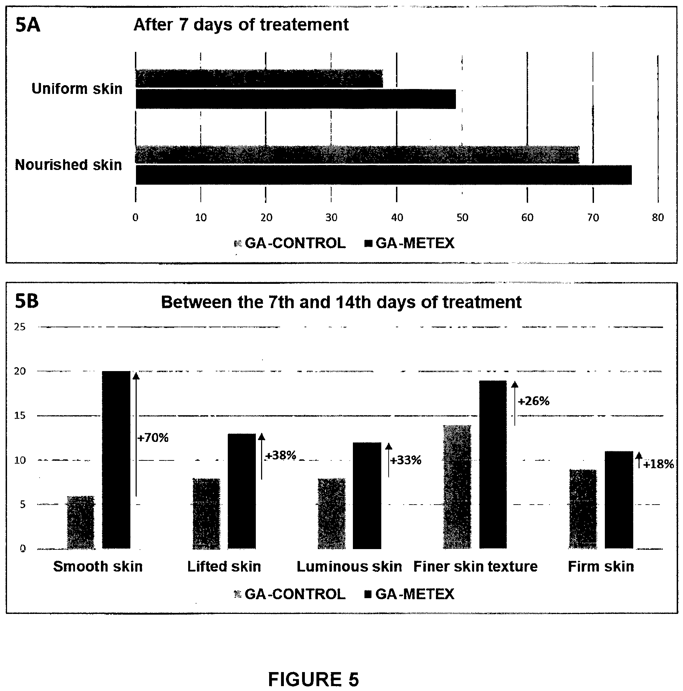

From the first week of use, both formulas received a good level of overall satisfaction from consumers (average rating 6.6/9). This result is explained by the effects perceived at the 7th day of application (**Figure 5A**).

Between the 7th and the 14th day, a significant increase in user satisfaction is observed (**Figure 5B**) for the treatment with GA-METEX.

The positive evolution between the 7th and the 14th day proves the effectiveness of the glycolic acid on improving skin quality. The changes in skin quality between the two creams are clearly in favor of GA-METEX cream.

EP 4 631 928 A1

## 3. Conclusion

All results obtained show the effect of the two tested glycolic acids on hydration, TEWL and sebum parameters. However, the effects are much more significant with GA-METEX cream. It is interesting to note that the unique composition of GA-METEX with compounds belonging to Natural Moisturizing Factors that allows the maintenance a good quality of the barrier function than the composition of GA-CONTROL.

The usage test makes it possible to demonstrate the effectiveness of the 2 formulas on markers of skin aging that cover the following items: nourished skin, uniform skin, smooth skin, luminous skin, finer skin texture, firm skin. The changes in skin quality between the two creams are clearly in favor of GA-METEX cream. We thus note, for example, that the percentage of satisfaction for the smoother skin item increases by 20 points for GA-METEX cream versus 6 points for GA-CONTROL cream, a satisfaction gain of 70%. The unique composition of GA-METEX obtained by the process of invention, in particular formaldehyde and methoxy acetic acid free, contributes to the results observed.

## REFERENCES

**PATENTS**

WO2007/140816
WO2007/141316
WO2010/108909
WO2011/036213
WO2011/157728
WO2012/025780
CN105647844A
CN106011185A
WO2016/079440
WO2018/138240
WO2013/050659
WO2014/162063
WO2016/193540
FR2974803B1
FR2974804B1
WO2006/069129
EP1947079B1
WO2006/016705
WO2016/044713
WO9709027
WO2016120796
WO2019133237
WO2021226632

**SCIENTIFIC LITTERATURE**

Anderson, (1946), Proc. Natl. Acad. Sci. USA., 32:120-128. Schaefer et al., (1999), Anal. Biochem. 270: 88-96. Meile et al., (2001), J Bacteriol., 183 (9), 2929-2936 Posthuma et al., (2002), Appl. Environ. Microbiol., 68(9): 831-837.

## Claims

1. Process for the extraction and purification of glycolic acid from a fermentation broth, comprising at least the successive steps of:

   a) Filtrating the fermentation broth,
   b) Passing said filtered fermentation broth through ion exchange resins, to obtain a glycolic acid solution,
   c) Concentrating said glycolic acid solution with successive steps of evaporation and nanofiltration, to obtain a concentrated solution,

d) Crystallizing glycolic acid by cooling of the concentrated solution.

2. Process for the extraction and purification of glycolic acid according to claim 1, further comprising a step of clarification of the fermentation broth, carried out by ultrafiltration before step (a).

3. Process for the extraction and purification of glycolic acid according to claim 2, wherein the clarification step is performed with a ceramic or organic membrane.

4. Process for the extraction and purification of glycolic acid according to anyone of claims 1 to 3, wherein step (a) is performed by nanofiltration.

5. Process for the extraction and purification of glycolic acid according to anyone of claims 1 to 4, wherein in step (b), the filtered fermentation broth is successively passed through a strong cation exchange resin and a weak anion exchange resin.

6. Process for the extraction and purification of glycolic acid according to anyone of claims 1 to 5, wherein in step (c), the glycolic acid solution is successively:

   - concentrated by water evaporation,
   - nanofiltered, and
   - concentrated with a rectification column, including addition of stripping water.

7. Process for the extraction and purification of glycolic acid according to anyone of claims 1 to 6, wherein in step (d), the temperature of the concentrated solution is lowered to a temperature ranging from 5°C to -25°C, preferably ranging from -10°C to -20°C.

8. Process for the extraction and purification of glycolic acid according to anyone of claims 1 to 7, further comprising a step (e) of dissolution of glycolic acid crystals into an aqueous buffer, and of polishing with an absorbent, preferably activated carbon.

9. Composition of bio-sourced glycolic acid, comprising glycolic acid, succinic acid and uracil.

10. Composition according to claim 9, wherein the glycolic acid is present in a percentage of at least 65% w/w, preferably at least 70% w/w.

11. Composition according to anyone of claims 9 to 10, wherein the succinic acid concentration ranges from 500 and 5000 ppm, preferably from 1000 and 3000 ppm, and uracil concentration is lower than 500 ppm, preferably lower than 100 ppm.

12. Composition according to anyone of claims 9 to 11, wherein said composition further comprises glyoxylic acid, in a concentration lower than 200 ppm, preferably lower than 150 ppm.

13. Composition according to anyone of claims 9 to 12, wherein said composition is obtained by the process according to anyone of claims 1 to 8.

14. Use of a composition according to anyone of claims 9 to 13 for cosmetic applications, in particular as a moisturizing agent or as an exfoliating agent.

15. Cosmetic composition comprising, as an active agent, a composition according to anyone of claims 9 to 13.

**FIGURE 1**

FIGURE 2

FIGURE 3

**4A    Sebum secretion: immediate effect**

GA-METEX: + 52,4

GA-CONTROL: + 42

T0    T 10 min        T0    T 10 min

GA-METEX          GA-CONTROL

**4B    Sebum secretion at 14 days**

GA-METEX: + 23,6

GA-CONTROL: + 17,7

T0    T 14 jours        T0    T 14 jours

GA-METEX          GA-CONTROL

FIGURE 4

**5A**     After 7 days of treatement

Uniform skin

Nourished skin

0    10    20    30    40    50    60    70    80

GA-CONTROL    GA-METEX

**5B**     Between the 7th and 14th days of treatment

+70%    +38%    +33%    +26%    +18%

Smooth skin    Lifted skin    Luminous skin    Finer skin texture    Firm skin

GA-CONTROL    GA-METEX

FIGURE 5

Europäisches
Patentamt

European
Patent Office

Office européen
des brevets

# EUROPEAN SEARCH REPORT

**Application Number**

EP 24 31 5143

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | EP 1 947 079 B1 (MITSUI CHEMICALS INC [JP]) 8 May 2019 (2019-05-08) * examples 2,3,5-8 * * paragraph [0001] - paragraph [0002] * | 1-15 | INV. C07C51/47 C07C51/43 C07C59/06 |
| X,D | FR 2 974 804 A1 (ROQUETTE FRERES [FR]) 9 November 2012 (2012-11-09) | 1-15 | |
| Y | * page 11, paragraph 1; claims 1-5 * * page 16, paragraph 1 * | 1-15 | |
| Y | JP 2010 126512 A (TORAY INDUSTRIES) 10 June 2010 (2010-06-10) * claims 1, 6, 9 * | 1-15 | |
| Y | CN 1 789 228 A (SHANGHAI PESTICIDE RES INST [CN]) 21 June 2006 (2006-06-21) * claims 1, 4 * | 1-15 | |
| X | US 2010/285077 A1 (LINTNER KARL [FR] ET AL) 11 November 2010 (2010-11-11) * paragraph [0115] * | 9-15 | |

**TECHNICAL FIELDS SEARCHED (IPC)**

C07C

The present search report has been drawn up for all claims

| Place of search | Date of completion of the search | Examiner |
|---|---|---|
| The Hague | 23 September 2024 | Öztürk Düzenli, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 31 5143

This annex lists the patent family members relating to the patent documents cited in the above-mentioned European search report.
The members are as contained in the European Patent Office EDP file on
The European Patent Office is in no way liable for these particulars which are merely given for the purpose of information.

23-09-2024

| Patent document cited in search report | | Publication date | Patent family member(s) | | Publication date |
|---|---|---|---|---|---|
| EP 1947079 | B1 | 08-05-2019 | CN | 101277920 A | 01-10-2008 |
| | | | EP | 1947079 A1 | 23-07-2008 |
| | | | JP | WO2007049707 A1 | 30-04-2009 |
| | | | KR | 20080061404 A | 02-07-2008 |
| | | | US | 2010022740 A1 | 28-01-2010 |
| | | | US | 2014018514 A1 | 16-01-2014 |
| | | | WO | 2007049707 A1 | 03-05-2007 |
| FR 2974804 | A1 | 09-11-2012 | FR | 2974804 A1 | 09-11-2012 |
| | | | WO | 2012153043 A1 | 15-11-2012 |
| JP 2010126512 | A | 10-06-2010 | NONE | | |
| CN 1789228 | A | 21-06-2006 | NONE | | |
| US 2010285077 | A1 | 11-11-2010 | CN | 101854907 A | 06-10-2010 |
| | | | EP | 2209460 A2 | 28-07-2010 |
| | | | FR | 2920967 A1 | 20-03-2009 |
| | | | JP | 5209724 B2 | 12-06-2013 |
| | | | JP | 2010539157 A | 16-12-2010 |
| | | | US | 2010285077 A1 | 11-11-2010 |
| | | | WO | 2009034537 A2 | 19-03-2009 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007140816 A
- WO 2007141316 A
- WO 2010108909 A
- WO 2011036213 A
- WO 2011157728 A
- WO 2012025780 A
- CN 105647844 A
- CN 106011185 A
- WO 2016079440 A
- WO 2018138240 A
- WO 2013050659 A
- WO 2014162063 A

- WO 2016193540 A
- FR 2974803 B1
- FR 2974804 B1
- WO 2006069129 A
- EP 1947079 B1
- WO 2006016705 A
- WO 2016044713 A
- WO 9709027 A
- WO 2016120796 A
- WO 2019133237 A
- WO 2021226632 A

**Non-patent literature cited in the description**

- *CHEMICAL ABSTRACTS*, 79-14-1
- *CHEMICAL ABSTRACTS*, 502-97-6
- *CHEMICAL ABSTRACTS*, 110-99-6
- **ANDERSON**. *Proc. Natl. Acad. Sci. USA.*, 1946, vol. 32, 120-128

- **SCHAEFER et al.** *Anal. Biochem.*, 1999, vol. 270, 88-96
- **MEILE et al.** *J Bacteriol.*, 2001, vol. 183 (9), 2929-2936
- **POSTHUMA et al.** *Appl. Environ. Microbiol.*, 2002, vol. 68 (9), 831-837